(19) **Europäisches Patentamt / European Patent Office / Office européen des brevets**

(11) **EP 3 662 813 B1**

(12) **EUROPEAN PATENT SPECIFICATION**

(45) Date of publication and mention
of the grant of the patent:
**24.11.2021 Bulletin 2021/47**

(51) Int Cl.:
*A61B 3/10* *(2006.01)*    *A61B 3/113* *(2006.01)*
*G06F 3/01* *(2006.01)*    *A61B 5/297* *(2021.01)*
*A61B 5/11* *(2006.01)*    *A61B 5/00* *(2006.01)*

(21) Application number: **18843467.4**

(22) Date of filing: **10.08.2018**

(86) International application number:
**PCT/JP2018/030056**

(87) International publication number:
**WO 2019/031600 (14.02.2019 Gazette 2019/07)**

(54) **WEARABLE DEVICE-COMPATIBLE ELECTROOCULOGRAPHY DATA PROCESSING DEVICE, SPECTACLE-TYPE WEARABLE DEVICE PROVIDED WITH SAME, AND WEARABLE DEVICE-COMPATIBLE ELECTROOCULOGRAPHY DATA PROCESSING METHOD**

MIT EINEM AM KÖRPER TRAGBAREN GERÄT KOMPATIBLES ELEKTROOKULOGRAFISCHES DATENVERARBEITUNGSGERÄT, MIT DIESEM AUSGESTATTETES AM KÖRPER TRAGBARES BRILLENARTIGES GERÄT UND MIT EINEM AM KÖRPER TRAGBAREN GERÄT KOMPATIBLES ELEKTROOKULOGRAFISCHES DATENVERARBEITUNGSVERFAHREN

DISPOSITIF DE TRAITEMENT DE DONNÉES D'ÉLECTROOCULOGRAPHIE COMPATIBLE AVEC UN DISPOSITIF PORTABLE, DISPOSITIF PORTABLE DE TYPE LUNETTES POURVU DE CELUI-CI, ET PROCÉDÉ DE TRAITEMENT DE DONNÉES D'ÉLECTROOCULOGRAPHIE COMPATIBLE AVEC UN DISPOSITIF PORTABLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.08.2017 JP 2017155948**

(43) Date of publication of application:
**10.06.2020 Bulletin 2020/24**

(73) Proprietor: **Yamaha Hatsudoki Kabushiki Kaisha Iwata-shi, Shizuoka 438-8501 (JP)**

(72) Inventors:
- **MORISHIMA, Keisuke**
  **Iwata-shi**
  **Shizuoka 438-8501 (JP)**
- **YAMANAKA, Kimihiro**
  **Kobe-shi**
  **Hyogo 658-8501 (JP)**
- **CHIHARA, Takanori**
  **Kanazawa-shi**
  **Ishikawa 920-1192 (JP)**

(74) Representative: **Zimmermann, Tankred Klaus et al Schoppe, Zimmermann, Stöckeler Zinkler, Schenk & Partner mbB Patentanwälte Radlkoferstrasse 2 81373 München (DE)**

(56) References cited:
**JP-A- 2014 124 308    JP-A- 2015 202 197
JP-A- 2017 094 121    US-A1- 2004 070 729
US-A1- 2017 150 897**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

EP 3 662 813 B1

- P Wiebe ET AL: "BIOSIGNALVERARBEITUNG DES MENSCHLICHEN ELEKTROOKULOGRAMMS (EOG) ZUR STEUERUNG VON COMPUTER-EINGABEMEDIEN F?R GEL?HMTE MENSCHEN", Biomedizinische Technik/Biomedical Engineering, 17 July 2009 (2009-07-17), pages 184-185, XP55358513, DOI: 10.1515/bmte.2000.45.s1.184 Retrieved from the Internet: URL:http://www.degruyter.com/dg/viewarticl e.fullcontentlink:pdfeventlink/$002fj$002f bmte.2000.45.issue-s1$002fbmte.2000.45.s1. 184$002fbmte.2000.45.s1.184.pdf?format=INT &t:ac=j$002fbmte.2000.45.issue-s1$002fbmte .2 000.45.s1.184$002fbmte.2000.45.s1.184.xm l

## Description

TECHNICAL FIELD

The present teaching relates to a wearable-device-compatible-electrooculography-data-processing device, a spectacle-type-wearable device provided with the wearable-device-compatible-electrooculography-data-processing device, and a wearable-device-compatible-electrooculography-data-processing method.

BACKGROUND ART

Methods in which a driving margin is evaluated using an eye potential have been examined. For example, in Non-patent Document 1, a method in which a driving margin is evaluated using an eye potential is disclosed. In Non-patent Document 1, the driving margin is evaluated using, as an eye motion-related parameter calculated from the eye potential, a blink rate, an eye motion norm, an eye motion ratio, and an eye motion time.

On the other hand, electrooculography measuring devices which measure the eye potential have been examined. As an electrooculography measuring device, a wearable device which is free from strange feeling when a user wears the wearable device and has been achieved by the progress of the sensing technology, has been proposed. For example, in Patent Document 1, as a spectacle-type-electrooculography-measuring device, an eyewear including a bioelectrode used for acquiring an eye potential signal is disclosed. Note that, in the eyewear disclosed in Patent Document 1, the bioelectrode is provided in each of a pair of nose pads, and a bridge portion.

CITATION LIST

PATENT DOCUMENT

Patent Document 1: Japanese Patent Application Publication No. 2017-070602 NON-PATENT DOCUMENT Patent Document 2: United States Patent Application Publication No. US 2017/150897 A1 Patent Document 3: United States Patent Application Publication No. US 2004/0070729 A1

Non-patent Document 1: "Evaluation of Driver's Safety Margin Using Parameters Related to EOG and Head Movements for Wearable Device," Soichiro Yokoo and six others, September 6, 2016, No. 3D2-2 (CD-ROM), Human Interface Society

SUMMARY OF INVENTION

TECHNICAL PROBLEM

As described above, in the wearable device which measures the eye potential, a user's wearing position is different from a reference wearing position in design of the wearable device in some cases. There is also a probability that the wearing position of the wearable device changes due to vibration or the like after a user wears the wearable device. As described above, in the wearable device, the reference wearing position and the user's wearing position are different in some cases.

As described above, when the reference wearing position and the user's wearing position are different in the wearable device, a position of a bioelectrode that measures an eye potential of the user is different from a reference position. Therefore, data of the eye potential that is measured using the above described bioelectrode differs depending on the wearing position of the wearable device.

As a result, eye motion data acquired from data of the measured eye potential differs depending on the wearing position of the wearable device.

It is an object of the present teaching to provide a wearable-device-compatible-electrooculography-data-processing device that can cope with a problem of a wearable device in which, depending on a position of an electrode that measures an eye potential while the wearable device is worn, a measured value of the eye potential differs.

SOLUTION TO PROBLEM

The present inventors conducted examinations on change of eye motion data due to change of a position of an electrode that measures an eye potential.

The present inventors considered a method in which change of a position of an electrode that measures an eye potential is allowed, not a method in which a position of an electrode that measures an eye potential is adjusted such that data of the eye potential does not change.

Through examinations on the method in which change of the position of the electrode is allowed, the present inventors focused on acquiring eye movement amount continuity data related to an amount of eye movement that is one of eye motions, based on eye potential continuity data acquired by continuously measuring the eye potential.

It has been found that the change of the position of the electrode when a user uses the wearable device can be detected using the continuously acquired eye movement amount continuity data.

It has also been found that the change of the position of the electrode can also be detected using the continuously acquired eye potential continuity data.

If the change of the position of the electrode can be detected in the above described manner, the problem that arises when the position of the electrode changes can be coped with.

Specifically, the present inventors arrived at the following structure.

A wearable-device-compatible-electrooculography-data-processing device according to an embodiment of the present teaching continuously acquires wearable-electrooculography-continuity data from an electrooculography measuring device provided in a wearable device that a user wears, the electrooculography measuring device configured to continuously measure an eye potential of the user to provide the wearable-electrooculography-continuity data based on the continuously measured eye potential, and wherein the wearable-device-compatible-electrooculography-data-processing device acquires, based on the wearable-electrooculography-continuity data continuously acquired while the wearable device is used, continuous wearable-eye-movement-amount-continuity data related to an eye movement amount of the user while the wearable device is used.

In this structure, while the user uses the wearable device, the continuous wearable-eye-movement-amount-continuity data related to the eye movement amount can be continuously acquired. The wearable-eye-movement-amount-continuity data is continuously acquired, and thereby, it is understood that the wearable-eye-movement-amount-continuity data has changed with respect to data obtained by the electrooculography measuring device from a reference wearing position in design of the wearable device. Based on this change of the data, at least one of the wearable-electrooculography-continuity data or the wearable-eye-movement-amount-continuity data can be corrected or calibrated.

Thus, a problem of the wearable device in which a position of the electrooculography measuring device that measures the eye potential while the wearable device is worn changes can be coped with.

Accordingly, the eye potential measured by the electrooculography measuring device provided in the wearable device that the user wears, not by an electrooculography measuring device that is used in a laboratory and is configured such that an electrode is directly attached to the skin, can be used in evaluation of a driving margin or the like.

According to another aspect, the wearable-device-compatible-electrooculography-data-processing device according to the present teaching preferably has a structure below. The wearable-device-compatible-electrooculography-data-processing device corrects at least one of the wearable-electrooculography-continuity data or the wearable-eye-movement-amount-continuity data, based on at least one of the wearable-electrooculography-continuity data or the wearable-eye-movement-amount-continuity data.

According to this structure, at least one of the wearable-electrooculography-continuity data or the wearable-eye-movement-amount-continuity data can be corrected using at least one of the wearable-electrooculography-continuity data or the wearable-eye-movement-amount-continuity data.

Thus, even when the position of the electrooculography measuring device that measures the eye potential changes while the wearable device is worn, at least one of the wearable-electrooculography-continuity data or the wearable-eye-movement-amount-continuity data can be corrected.

A face shape differs for each user, and therefore, even when the eye potential measured by the electrooculography measuring device of the wearable device differs for each user, at least one of the wearable-electrooculography-continuity data or the wearable-eye-movement-amount-continuity data can be corrected.

Furthermore, even when the eye potential measured by the electrooculography measuring device of the wearable device changes due to change of skin impedance of the same user, at least one of the wearable-electrooculography-continuity data or the wearable-eye-movement-amount-continuity data can be corrected.

According to another aspect, the wearable-device-compatible-electrooculography-data-processing device according to the present teaching preferably has a structure below. The wearable-device-compatible-electrooculography-data-processing device is configured such that the electrooculography measuring device continuously measures eye potentials of the user at a plurality of positions. For each of the plurality of positions, the wearable-device-compatible-electrooculography-data-processing device continuously acquires a plurality of wearable-electrooculography-continuity data, based on the eye potentials at the plurality of positions, the eye potentials having been continuously measured by the electrooculography measuring device.

According to this structure, the wearable-eye-movement-amount-continuity data can be more accurately acquired by continuously measuring the plurality of eye potentials and acquiring the plurality of wearable-electrooculography-continuity data, based on the measured eye potentials at the plurality of positions.

For example, movement in eye motion in an up-down direction and a left-right direction can be acquired and also the eye movement amount continuity data can be more accurately acquired by continuously measuring two eye potentials, that is, eye potentials of the right eye and the left eye.

According to another aspect, the wearable-device-compatible-electrooculography-data-processing device according to the present teaching preferably has a structure below. The wearable-eye-movement-amount-continuity data is data related to a magnitude of the eye potential.

According to this structure, the eye movement amount continuity data is related to the magnitude of the eye potential, and therefore, the wearable-eye-movement-amount-continuity data can be more easily acquired.

According to another aspect, the wearable-device-compatible-electrooculography-data-processing device according to the present teaching preferably has a structure below. The wearable-device-compatible-electrooculography-data-processing device continuously acquires wearable-head-motion-continuity data of the user from a head-motion-measuring device provided in the wearable device, wherein the wearable-device-compatible-electrooculography-data-processing device acquires wearable head movement amount continuity data from the continuously acquired wearable-head-motion-continuity data, and wherein the wearable-device-compatible-electrooculography-data-processing device acquires wearable-visual-line-movement-amount-continuity data from the wearable-eye-movement-amount-continuity data and the wearable head movement amount continuity data.

According to this structure, in addition to the eye motion, the head motion can be taken into consideration, and therefore, the wearable-visual-line-movement-amount-continuity data can be more accurately acquired.

According to another aspect, the wearable-device-compatible-electrooculography-data-processing device according to the present teaching preferably has a structure below. The wearable-device-compatible-electrooculography-data-processing device acquires, from the wearable-electrooculography-continuity data and the wearable-head-motion-continuity data, continuous wearable-head-eye-coordinated-motion-continuity data related to a coordinated motion of eye motion and head motion while the wearable device is used, and wherein the wearable-device-compatible-electrooculography-data-processing device outputs the wearable-visual-line-movement-amount-continuity data that corresponds to the predetermined wearable-head-eye-coordinated-motion-continuity data.

According to this structure, the wearable-visual-line-movement-amount-continuity data that corresponds to the predetermined wearable-head-eye-coordinated-motion-continuity data can be output. Thus, the output wearable-visual-line-movement-amount-continuity data can be used also in evaluation of a driving margin.

According to another aspect, the wearable-device-compatible-electrooculography-data-processing device according to the present teaching preferably has a structure below. The wearable-device-compatible-electrooculography-data-processing device includes: a wearable-electrooculography-continuity-data-acquisition section configured to continuously acquire the wearable-electrooculography-continuity data from the electrooculography measuring device; and a wearable-eye-movement-amount-continuity-data-acquisition section configured to acquire, based on the wearable-electrooculography-continuity data acquired by the wearable-electrooculography-continuity-data-acquisition section, while the wearable device is used, the continuous wearable-eye-movement-amount-continuity data.

A spectacle-type-wearable device according to an embodiment of the present teaching includes the wearable-device-compatible-electrooculography-data-processing device having each of the above described structures.

According to this structure, the user wears the spectacle-type-wearable device, and thereby, the continuous wearable-eye-movement-amount-continuity data related to the eye movement amount of the user can be acquired by the spectacle-type-wearable device.

A wearable-device-compatible-electrooculography-data-processing method according to an embodiment of the present teaching includes continuously acquiring wearable-electrooculography-continuity data from an electrooculography measuring device provided in a wearable device that a user wears and that is configured to continuously measure an eye potential of the user to provide the wearable-electrooculography-continuity data based on the continuously measured eye potential, and acquiring, based on the wearable-electrooculography-continuity data continuously acquired while the wearable device is used, continuous wearable-eye-movement-amount-continuity data related to an eye movement amount of the user while the wearable device is used.

According to another aspect, the wearable-device-compatible-electrooculography-data-processing method according to the present teaching preferably has the structure below. In the wearable-device-compatible-electrooculography-data-processing method, at least one of the wearable-electrooculography-continuity data or the wearable-eye-movement-amount-continuity data is corrected based on at least one of the wearable-electrooculography-continuity data or the wearable-eye-movement-amount-continuity data.

According to another aspect, the wearable-device-compatible-electrooculography-data-processing method according to the present teaching preferably has the structure below. The electrooculography measuring device continuously measures eye potentials at a plurality of positions. In the wearable-device-compatible-electrooculography-data-processing method, for each of the plurality of positions, a plurality of wearable-electrooculography-continuity data are continuously acquired based on the eye potentials at the plurality of positions, the eye potentials having been continuously measured by the electrooculography measuring device.

According to another aspect, the wearable-device-compatible-electrooculography-data-processing method according to the present teaching preferably has the structure below. The wearable-eye-movement-amount-continuity data is data related to a magnitude of the eye potential.

According to another aspect, the wearable-device-compatible-electrooculography-data-processing method according to the present teaching preferably has the structure below. The wearable-device-compatible-electrooculography-data-processing method further includes continuously acquiring wearable-head-motion-continuity data of the user from a head-motion-measuring device provided in the wearable device, acquiring wearable head movement amount continuity data from the continuously acquired wearable-head-motion-continuity data, and acquiring wearable-visual-line-movement-amount-continuity data from the wearable-eye-movement-amount-continuity data and the wearable head movement amount continuity data.

According to another aspect, the wearable-device-compatible-electrooculography-data-processing method according to the present teaching preferably has the structure below. The wearable-device-compatible-electrooculography-data-processing method further includes acquiring, from the wearable-electrooculography-continuity data and the wearable-head-motion-continuity data, continuous wearable-head-eye-coordinated-motion-continuity data related to a coordinated motion of eye motion and head motion while the wearable device is used, and outputting the wearable-visual-line-movement-amount-continuity data that corresponds to the predetermined wearable-head-eye-coordinated-motion-continuity data.

The terminology used herein is for the purpose of describing particular embodiments only and is not intended to be limiting of the present teaching.

As used herein, the term "and/or" includes any and all combinations of one or more of the associated listed items.

It will be further understood that the terms "including", "comprising" or "having" and variations thereof when used in this specification, specify the presence of stated features, steps, elements, components, and/or their equivalents, but do not preclude the presence or addition of one or more other steps, operations, elements, components, and/or groups thereof.

As used herein, the terms "mounted," "connected," "coupled," and/or their equivalents thereof are used broadly and encompass both "direct and indirect" mounting, connecting, and coupling. Furthermore, "connected" and "coupled" are not restricted to physical or mechanical connections or couplings, and can include direct or indirect connections or couplings.

Unless otherwise defined, all terms (including technical and scientific terms) used herein have the same meaning as commonly understood by one having ordinary skill in the art to which the present teaching belongs.

It will be further understood that terms, such as those defined in commonly used dictionaries, should be interpreted as having a meaning that is consistent with their meaning in the context of the relevant art and present disclosure and will not be interpreted in an idealized or overly formal sense unless expressly so defined herein.

In describing the present teaching, it will be understood that a number of techniques and steps are disclosed. Each of these has individual benefit and each can also be used in conjunction with one or more, or in some cases all, of the other disclosed techniques.

Accordingly, for the sake of clarity, this description will refrain from repeating every possible combination of the individual steps in an unnecessary fashion. Nevertheless, the specification and claims should be read with the understanding that such combinations are entirely within the scope of the present teaching.

Embodiments of a wearable-device-compatible-electrooculography-data-processing device according to the present teaching will be described herein.

In the following description, numerous specific examples are set forth in order to provide a thorough understanding of the present teaching. It will be evident, however, to one skilled in the art that the present teaching may be practiced without these specific examples.

The present disclosure is to be considered as an exemplification of the teaching, and is not intended to limit the teaching to the specific embodiments illustrated by the figures or description below.

[Continuously Acquire Wearable-Electrooculography-Continuity Data]

As used herein the term "continuously acquiring wearable-electrooculography-continuity data" includes not only acquiring the wearable-electrooculography-continuity data from a measured eye potential by an arithmetic operation, but also acquiring the wearable-electrooculography-continuity data by transforming the measured eye potential using a map set in advance.

[Continuously Acquire Wearable-Eye-Movement-Amount-Continuity Data]

As used herein the term "continuously acquiring wearable-eye-movement-amount-continuity data" includes not only acquiring the wearable-eye-movement-amount-continuity data from the wearable-electrooculography-continuity data that has been already acquired by an arithmetic operation but also acquiring the wearable-eye-movement-amount-continuity data by transforming the wearable-electrooculography-continuity data that has been already acquired using a map set in advance.

[Eye Motion]

As used herein, the term "eye motion" means motion of the eyes relative to the head. That is, the eye motion means movement of the eyes relative to the head. The eye motion includes saccade (saccadic eye motion) in which, for example, an absolute value of an eye rotation angular velocity is a threshold $\alpha$ (for example, 30 deg/sec) or more and an absolute value of a motion amount obtained by integrating the rotation angular velocity is a threshold $\beta$ (for example, 3.5 deg) or more.

[Head Motion]

As used herein, the term "head motion" means motion of a head when the head moves or rotates in absolute coordinates in an up-down direction and a left-right direction. That is, the head motion includes motion of the head when the head moves or rotates relative to a body, motion of the head when the head and the body move or rotate together, and motion of the head when the head and the body separately move or rotate.

[Effective Visual Field]

As used herein, the term "effective visual field" is a range in which a subject can collect visual information effectively used in an action of capturing a visual object in a field of vision.

[Continuously Acquire Wearable-Head-Motion-Continuity Data]

As used herein the term "continuously acquiring wearable-head-motion-continuity data" includes not only acquiring the wearable-head-motion-continuity data from information of the head-motion-measuring device that has been measured by an arithmetic operation but also acquiring the wearable-head-motion-continuity data by transforming the measured information of the head-motion-measuring device using a map set in advance.

[Acquire Continuous Wearable-Head-Eye-Coordinated-Motion-Continuity Data Related to Coordinated Motion of Eye Motion and Head Motion While Wearable Device Is Used]

As used herein the term "acquiring continuous wearable-head-eye-coordinated-motion-continuity data related to a coordinated motion of eye motion and head motion while the wearable device is used" includes not only acquiring the wearable-head-eye-coordinated-motion-continuity data from the wearable-electrooculography-continuity data and the wearable-head-motion-continuity data that have been already acquired by an arithmetic operation but also acquiring the wearable-head-eye-coordinated-motion-continuity data by transforming the wearable-electrooculography-continuity data and the wearable-head-motion-continuity data that have been already acquired using a map set in advance.

[Coordinated Motion of Eye Motion and Head Motion]

As used herein, the term "coordinated motion of eye motion and head motion" is motion including at least one of eye motion and head motion in a viewing action when a user views a visual object.

[Visual-Line-Movement Amount]

As used herein, the term "visual-line-movement amount" is a value obtained by summing an eye movement amount that is a movement amount of eyes and a head movement amount that is a movement amount of a head.

[Wearable Device]

As used herein the term "wearable device" means a device provided in a wearing tool, such as glasses, sunglasses, goggles, a head mount display, or the like, the wearing tool being worn by a user. Therefore, in embodiments, as an example of the wearable device, a spectacle-type-wearable device is described, but the wearing device is not limited thereto. The wearing device may have any structure which the user can wear and in which an electrooculography measuring device that measures an eye potential can be disposed.

ADVANTAGEOUS EFFECTS OF INVENTION

Based on the foregoing, a wearable-device-compatible-electrooculography-data-processing device that can cope with the problem of wearable devices in which, depending on a position of an electrode that measures an eye potential while the wearable device is worn, a measured value of the eye potential differs can be provided.

BRIEF DESCRIPTION OF DRAWINGS

[FIG. 1] FIG. 1 is a schematic view illustrating an overall structure of a spectacle-type-wearable device according to a first embodiment of the present teaching.
[FIG. 2] FIG. 2 is an explanatory view illustrating an overall structure of a wearable-device-compatible-electrooculography-data-processing device in the spectacle-type-wearable device according to the first embodiment of the present teaching.
[FIG. 3] FIG. 3 is a block diagram illustrating the overall structure of the wearable-device-compatible-electrooculography-data-processing device in the first embodiment of the present teaching.
[FIG. 4] FIG. 4 is a functional block diagram illustrating, as a functional block, a function based on a processing operation in the wearable-device-compatible-electrooculography-data-processing device illustrated in FIG. 3.
[FIG. 5] FIG. 5 is a flowchart illustrating an example of process steps until the wearable-device-compatible-electrooculography-data-processing device in the first embodiment acquires wearable-eye-movement-amount-continuity data.
[FIG. 6] FIG. 6 is a flowchart illustrating an example of process steps for correcting the wearable-eye-movement-amount-continuity data while the spectacle-type-wearable device is worn.
[FIG. 7] FIG. 7 is an explanatory chart illustrating an example of how the wearable-eye-movement-amount-continuity data is corrected.
[FIG. 8] FIG. 8 is an explanatory chart illustrating a state in which a coordinate system is formed using a median of the acquired wearable-eye-movement-amount-continuity data.

[FIG. 9] FIG. 9 is an explanatory chart illustrating a state in which correction has been performed such that an intersection of a vertical axis and a horizontal axis is an origin.

[FIG. 10] FIG. 10 is a schematic chart illustrating a state in which affine transformation has been performed such that the horizontal axis is horizontal and the vertical axis is vertical.

[FIG. 11] FIG. 11 is an explanatory chart illustrating a state in which the wearable-eye-movement-amount-continuity data has been corrected in each quadrant.

[FIG. 12] FIG. 12 is a flowchart illustrating steps for correction processing for wearable-eye-movement-amount-continuity data in a second embodiment.

[FIG. 13] FIG. 13 is an explanatory chart illustrating a relationship between the wearable-electrooculography-continuity data and time.

[FIG. 14] FIG. 14 is a flowchart illustrating steps for correction processing for the wearable-electrooculography-continuity data.

[FIG. 15] FIG. 15 is an explanatory chart illustrating the relationship between the wearable-electrooculography-continuity data and time.

[FIG. 16] FIG. 16 is a flowchart illustrating an example of correction processing for wearable-eye-movement-amount-continuity data in a third embodiment.

[FIG. 17] FIG. 17 is an explanatory chart illustrating an example of how the wearable-eye-movement-amount-continuity data is corrected.

[FIG. 18] FIG. 18 is an explanatory chart illustrating an example in which the wearable-eye-movement-amount-continuity data has been corrected.

[FIG. 19] FIG. 19 is views schematically illustrating an example of a viewing action.

[FIG. 20] FIG. 20 is a functional block diagram of a wearable-device-compatible-electrooculography-data-processing device in a fourth embodiment of the present teaching.

[FIG. 21] FIG. 21 is a flowchart illustrating an operation in the fourth embodiment.

[FIG. 22] FIG. 22 is a table illustrating a relationship between a measured value by an electrooculography measuring device and an eye potential in a vertical direction.

DESCRIPTION OF EMBODIMENTS

Embodiments of the present teaching will be described below.

Leftward, rightward, upward, downward, forward, and rearward directions in the embodiments of the present teaching are leftward, rightward, upward, downward, forward, and rearward directions, respectively, as viewed from a user wearing a spectacle-type-wearable device 1. An arrow L in the drawings denotes a leftward direction of the spectacle-type-wearable device 1, an arrow R in the drawings denotes a rightward direction of the spectacle-type-wearable device 1, an arrow U in the drawings denotes an upward direction of the spectacle-type-wearable device 1, an arrow D in the drawings denotes a downward direction of the spectacle-type-wearable device 1, an arrow F in the drawings denotes a forward direction of the spectacle-type-wearable device 1, and an arrow RR in the drawings denotes a rearward direction of the spectacle-type-wearable device 1.

[First Embodiment]

(Entire Structure of Spectacle-Type-Wearable Device)

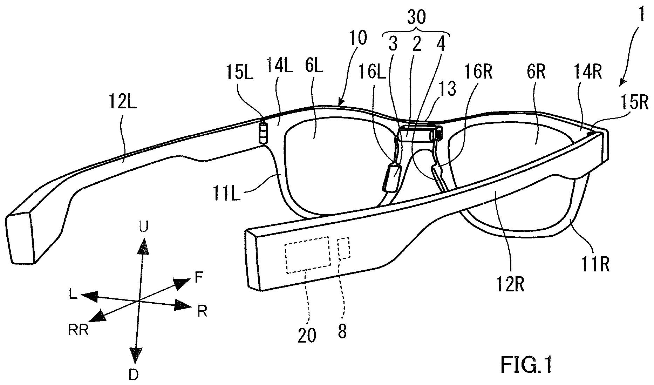

In FIG. 1, an overall structure of the spectacle-type-wearable device 1 according to an embodiment of the present teaching is illustrated. As illustrated in FIG. 1, the spectacle-type-wearable device 1 includes a frame 10, a left lens 6L, and a right lens 6R that are basic components of spectacles. The spectacle-type-wearable device 1 includes an electrooculography measuring device 30 that measures an eye potential of a user in a state in which the user wears the spectacle-type-wearable device 1 and a wearable-device-compatible-electrooculography-data-processing device 20 that processes electrooculography data measured by the electrooculography measuring device 30. Thus, the spectacle-type-wearable device 1 can measure the eye potential of the user and process the acquired electrooculography data in a state in which the user wears the spectacle-type-wearable device 1.

The frame 10 includes a left rim 11L, a right rim 11R, a bridge 13, a left temple 12L, a right temple 12R, a left nose pad 16L, and a right nose pad 16R.

The left rim 11L holds the left lens 6L. The right rim 11R holds the right lens 6R. The bridge 13 connects the left rim 11L and the right rim 11R. A left wraparound endpiece 14L is provided on a left side of the left rim 11L. A right wraparound endpiece 14R is provided on a right side of the right rim 11R.

The left temple 12L extends rearward from the left rim 11L. The left temple 12L is located at the left temple and left ear of the user in a state in which the user wears the spectacle-type-wearable device 1. The right temple 12R extends rearward from the right rim 11R. The right temple 12R is located at the right temple and right ear of the user in a state in which the user wears the spectacle-type-wearable device 1.

The left temple 12L is rotatably held with respect to the left wraparound endpiece 14L by a left hinge 15L provided in the left wraparound endpiece 14L. Similarly, the right temple 12R is rotatably held with respect to the right wraparound endpiece 14R by a right hinge 15R provided in the right wraparound endpiece 14R.

The left nose pad 16L is attached to the bridge 13 and is located on a right side of the left rim 11L when viewed from a rear side in a front-rear direction. The left nose pad 16L touches a left portion of the nose of the user in a state in which the user wears the spectacle-type-wearable device 1. The right nose pad 16R is attached to the bridge 13 and is located in a left side of the right rim 11R when viewed from the rear side in the front-rear direction. The right nose pad 16R touches a right portion of the nose of the user in a state in which the user wears the spectacle-type-wearable device 1.

The electrooculography measuring device 30 is provided in a central portion of the spectacle-type-wearable device 1 in a left-right direction. That is, the electrooculography measuring device 30 is located between the left rim 11L and right rim 11R when viewed from the rear side in the front-rear direction.

The electrooculography measuring device 30 includes a reference electrode 2, a left nose electrode 3, and a right nose electrode 4. The reference electrode 2 is provided in a rear portion of the bridge 13. The left nose electrode 3 is provided in the left nose pad 16L. The right nose electrode 4 is provided in the right nose pad 16R.

That is, in the spectacle-type-wearable device 1, the left nose electrode 3 and the right nose electrode 4 are provided under the reference electrode 2. Thus, when the user wears the spectacle-type-wearable device 1, the reference electrode 2 touches the middle of the eyebrows of the user, the left nose electrode 3 touches the left portion of the nose of the user, and the right nose electrode 4 touches the right portion of the nose of the user.

The wearable-device-compatible-electrooculography-data-processing device 20 is provided in the right temple 12R. The wearable-device-compatible-electrooculography-data-processing device 20 processes data of the eye potential output from the electrooculography measuring device 30. A detailed structure of the wearable-device-compatible-electrooculography-data-processing device 20 will be described later.

The spectacle-type-wearable device 1 of this embodiment further includes a power source 8 that supplies power to the electrooculography measuring device 30 and the wearable-device-compatible-electrooculography-data-processing device 20. The power source 8 is provided in the right temple 12R. That is, the wearable-device-compatible-electrooculography-data-processing device 20 and the power source 8 are disposed in line in the front-rear direction in the right temple 12R. The power source 8 is, for example, a secondary battery, such as a lithium ion battery or the like. Note that the power source 8 may have any structure in which the power source 8 can supply power to the electrooculography measuring device 30 and the wearable-device-compatible-electrooculography-data-processing device 20.

Power is supplied to the reference electrode 2, the left nose electrode 3, and the right nose electrode 4 by the power source 8, and thereby, the electrooculography measuring device 30 can measure an eye potential of the user wearing the spectacle-type-wearable device 1. The electrooculography measuring device 30 measures an eye potential of the left eye (which will be hereinafter referred to as a left eye potential) of the user by the reference electrode 2 and the left nose electrode 3, and measures an eye potential of the right eye (which will be hereinafter referred to as a right eye potential) of the user using the reference electrode 2 and the right nose electrode 4.

(Entire Structure of Wearable-Device-Compatible-Electrooculography-Data-Processing Device)

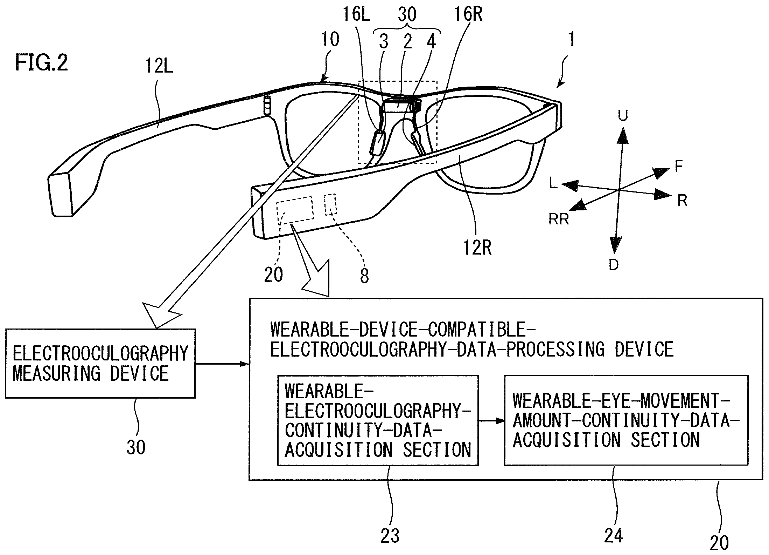

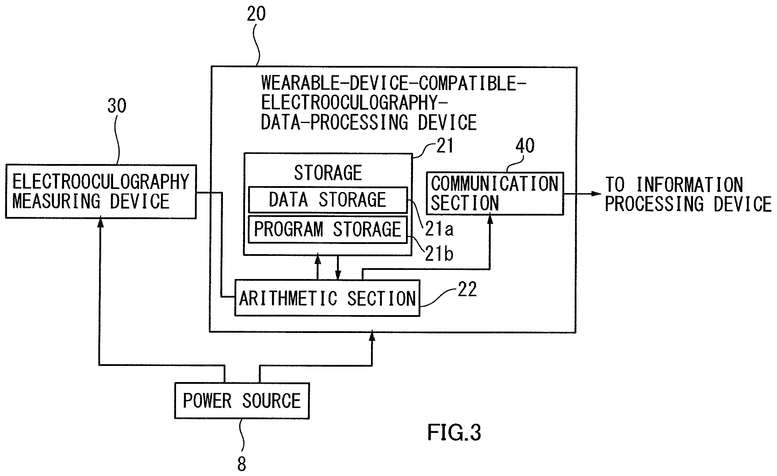

FIG. 2 is an explanatory view illustrating an overall structure of the wearable-device-compatible-electrooculography-data-processing device 20 in the spectacle-type-wearable device 1. FIG. 3 is a block diagram illustrating the overall structure of the wearable-device-compatible-electrooculography-data-processing device 20.

The wearable-device-compatible-electrooculography-data-processing device 20 continuously acquires, as wearable-electrooculography-continuity data, data of the eye potential continuously output from the electrooculography measuring device 30. The wearable-device-compatible-electrooculography-data-processing device 20 acquires, based on the wearable-electrooculography-continuity data acquired while the spectacle-type-wearable device 1 is used, wearable-eye-movement-amount-continuity data related to an eye movement amount while the spectacle-type-wearable device 1 is used.

Specifically, the wearable-device-compatible-electrooculography-data-processing device 20 includes a wearable-electrooculography-continuity-data-acquisition section 23 and a wearable-eye-movement-amount-continuity-data-acquisition section 24.

The wearable-electrooculography-continuity-data-acquisition section 23 continuously acquires, as the wearable-electrooculography-continuity data, the eye potential that is continuously output from the electrooculography measuring device 30 at a predetermined sampling frequency.

The wearable-eye-movement-amount-continuity-data-acquisition section 24 continuously acquires, based on the wearable-electrooculography-continuity data, the wearable-eye-movement-amount-continuity data related to the eye movement amount while the spectacle-type-wearable device 1 is used.

As will be described later, the wearable-device-compatible-electrooculography-data-processing device 20 operates in a manner described below.

The eye potential is continuously output from the electrooculography measuring device 30 that continuously measures the eye potential. The eye potential continuously output from the electrooculography measuring device 30 is continuously input to the wearable-device-compatible-electrooculography-data-processing device 20. The eye potential continuously input from the electrooculography measuring device 30 is continuously acquired as the wearable-electrooculography-continuity data at the predetermined sampling frequency in the wearable-electrooculography-continuity-data-acquisition section 23. The acquired wearable-electrooculography-continuity data is held in a storage 21, which will be described later.

The wearable-eye-movement-amount-continuity-data-acquisition section 24 acquires, based on the wearable-electrooculography-continuity data held in the storage 21, the continuous wearable-eye-movement-amount-continuity data related to the eye movement amount while the spectacle-type-wearable device 1 is used. The acquired wearable-eye-movement-amount-continuity data is held in the storage 21.

As described above, according to the wearable-device-compatible-electrooculography-data-processing device 20, the continuous wearable-eye-movement-amount-continuity data related to the eye movement amount can be continuously acquired while the user uses the spectacle-type-wearable device 1.

Next, an example of the structure of the wearable-device-compatible-electrooculography-data-processing device 20 will be described with reference to FIG. 3. FIG. 3 is a block diagram illustrating the overall structure of the wearable-device-compatible-electrooculography-data-processing device 20.

As illustrated in FIG. 3, the wearable-device-compatible-electrooculography-data-processing device 20 includes the storage 21 formed of a non-volatile memory or the like, an arithmetic section 22 including an internal memory (RAM) and a processor that are not illustrated in FIG. 3, and a communication section 40. As already described above, power is supplied to the electrooculography measuring device 30 and the wearable-device-compatible-electrooculography-data-processing device 20 from the power source 8.

The storage 21 includes a data storage 21a that stores various types of data and a program storage 21b that stores a program based on a processing algorithm or the like. The arithmetic section 22 performs various types of processing, based on the program stored in the program storage 21b.

The arithmetic section 22 operates based on the program stored in the program storage 21b and functions as the wearable-electrooculography-continuity-data-acquisition section 23. The arithmetic section 22 continuously fetches the left eye potential and the right eye potential continuously measured by the electrooculography measuring device 30 to the internal memory at the predetermined sampling frequency. The arithmetic section 22 calculates a vertical direction potential and a horizontal direction potential from the fetched left and right eye potentials and continuously stores, as the wearable-electrooculography-continuity data, the vertical direction potential and the horizontal direction potential that have been calculated in the data storage 21a of the storage 21.

The arithmetic section 22 operates based on the program stored in the program storage 21b and functions also as the wearable-eye-movement-amount-continuity-data-acquisition section 24. The arithmetic section 22 reads the wearable-electrooculography-continuity data stored in the data storage 21a of the storage 21 and calculates, based on the read wearable-electrooculography-continuity data, the continuous wearable-eye-movement-amount-continuity data related to the eye movement amount while the spectacle-type-wearable device 1 is used. The arithmetic section 22 stores the calculated continuous wearable-eye-movement-amount-continuity data in the data storage 21a.

The arithmetic section 22 functions also as a correction section 60 that corrects at least one of the wearable-electrooculography-continuity data or the continuous wearable-eye-movement-amount-continuity data that have been calculated in accordance with change of a position of each of the electrodes (the reference electrode 2, the left nose electrode 3, and the right nose electrode 4) of the electrooculography measuring device 30, as described later. The wearable-electrooculography-continuity data or the continuous wearable-eye-movement-amount-continuity data that have been corrected are stored in the data storage 21a.

The communication section 40 transmits the continuous wearable-eye-movement-amount-continuity data stored in the data storage 21a of the storage 21 to an external information processing device or the like via the arithmetic section 22. As the information processing device, for example, a mobile terminal, such as a smart phone or the like, a tablet terminal, such as an electronic device or the like that includes a touch panel mounted thereon, a PC terminal, or the like can be used. The information processing device may be a cloud server. In this case, the spectacle-type-wearable device may be configured to communicate with a mobile terminal, such as a smart phone or the like, via a cloud server that serves as the information processing device.

(Detailed Structure of Wearable-Device-Compatible-Electrooculography-Data-Processing Device)

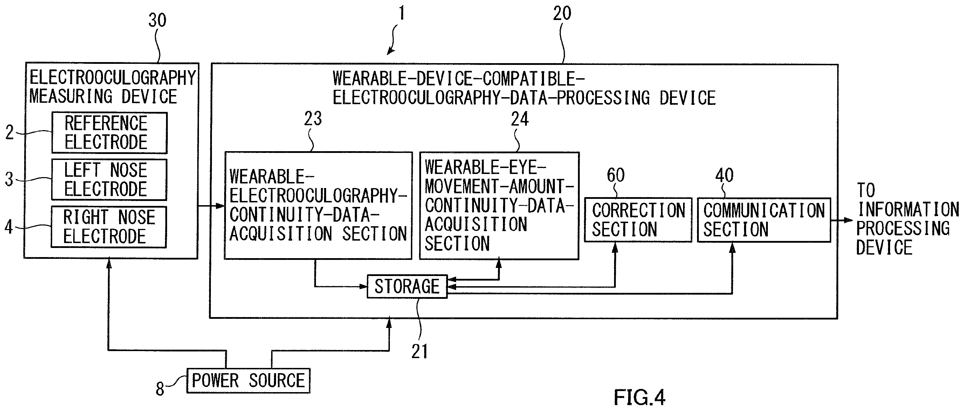

FIG. 4 is a functional block diagram illustrating, as a functional block, a function based on a processing operation in the wearable-device-compatible-electrooculography-data-processing device 20 illustrated in FIG. 3. With reference to FIG. 4, the wearable-device-compatible-electrooculography-data-processing device 20 will be described in detail.

The wearable-device-compatible-electrooculography-data-processing device 20 includes the wearable-electrooculography-continuity-data-acquisition section 23, the wearable-eye-movement-amount-continuity-data-acquisition section 24, the storage 21, the correction section 60, and the communication section 40. Power is supplied to the electrooculography measuring device 30 and the wearable-device-compatible-electrooculography-data-processing device 20 from the power source 8.

The electrooculography measuring device 30 continuously measures a left eye potential VL of the user by the reference electrode 2 and the left nose electrode 3 and continuously measures a right eye potential VR of the user by the reference electrode 2 and the right nose electrode 4. The electrooculography measuring device 30 continuously outputs the left eye potential VL and the right eye potential VR of the user, the left eye potential VL and the right eye potential VR having been continuously measured.

The wearable-electrooculography-continuity-data-acquisition section 23 continuously fetches the left eye potential VL and the right eye potential VR that have been continuously output from the electrooculography measuring device 30 at the predetermined sampling frequency. The wearable-electrooculography-continuity-data-acquisition section 23 continuously calculates a vertical direction potential Vv and a horizontal direction potential Vh from the left eye potential VL and the right eye potential VR that have been acquired and stores, as the wearable-electrooculography-continuity data, the vertical direction potential Vv and the horizontal direction potential Vh that have been continuously calculated in the storage 21.

The wearable-eye-movement-amount-continuity-data-acquisition section 24 reads the wearable-electrooculography-continuity data stored in the storage 21.

The wearable-eye-movement-amount-continuity-data-acquisition section 24 calculates, based on the wearable-electrooculography-continuity data including the vertical direction potential Vv and the horizontal direction potential Vh, the continuous wearable-eye-movement-amount-continuity data related to the eye movement amount while the spectacle-type-wearable device 1 is used and stores the calculated wearable-eye-movement-amount-continuity data in the storage 21.

As calibration, the correction section 60 corrects the continuous wearable-electrooculography-continuity data or the continuous wearable-eye-movement-amount-continuity data that have been calculated, as appropriate. The correction section 60 reads the continuous wearable-electrooculography-continuity data or the continuous wearable-eye-movement-amount-continuity data from the storage 21, performs necessary correction processing, and stores corrected data in the storage 21.

The wearable-eye-movement-amount-continuity data stored in the storage 21 is transmitted to an external information processing device 50 via the communication section 40.

(Process Steps of Wearable-Device-Compatible-Electrooculography-Data-Processing Device 20)

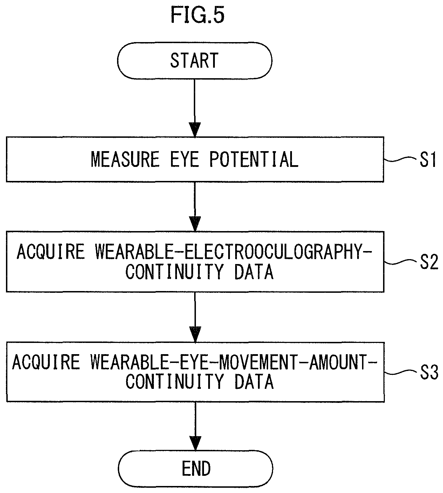

FIG. 5 is a flowchart illustrating an example of process steps until the wearable-device-compatible-electrooculography-data-processing device 20 in the first embodiment acquires the wearable-eye-movement-amount-continuity data. With reference to FIG. 5, a processing operation of the wearable-device-compatible-electrooculography-data-processing device 20 will be described.

In Step S1, the eye potential is measured by the electrooculography measuring device 30. Specifically, the left eye potential VL is measured from the left nose electrode 3 and the reference electrode 2. The right eye potential VR is measured from the right nose electrode 4 and the reference electrode 2.

In Step S2, the wearable-electrooculography-continuity-data-acquisition section 23 acquires the wearable-electrooculography-continuity data.

Specifically, the wearable-electrooculography-continuity-data-acquisition section 23 continuously fetches the left eye potential VL and the right eye potential VR measured in Step S1 and stores the left eye potential VL and the right eye potential VR in the storage 21. The wearable-electrooculography-continuity data-acquisition unit 23 calculates the vertical direction potential Vv using the left eye potential VL and the right eye potential VR stored in the storage 21, based on Expression 1 below. Note that, when the eyes move (rotate) upward from a front viewing state, Vv < 0 is established and, when the eyes move (rotate) downward from the front viewing state, Vv > 0 is established.

$$\text{(Expression 1)}$$

$$Vv = VR + VL$$

Similarly, the wearable-electrooculography-continuity-data-acquisition section 23 calculates and acquires the horizontal direction potential Vh, based on Expression 2 below. Note that, when the eyes move (rotate) rightward from the front viewing state, Vh < 0 is established and, when the eyes rotate leftward from the front viewing state, Vh > 0 is established.

$$\text{(Expression 2)}$$

$$Vh = VR - VL$$

As described above, the wearable-electrooculography-continuity-data-acquisition section 23 continuously calculates and acquires the vertical direction potential Vv and the horizontal direction potential Vh, based on the left eye potential VL and the right eye potential VR that have been continuously acquired. The vertical direction potential Vv and the horizontal direction potential Vh acquired by the wearable-electrooculography-continuity-data-acquisition section 23 are stored in the storage 21. Accordingly, the left eye potential VL and the right eye potential VR that have been continuously acquired, the vertical direction potential Vv, and the horizontal direction potential Vh are stored in the storage 21.

In Step S3, the wearable-eye-movement-amount-continuity-data-acquisition section 24 acquires the wearable-eye-movement-amount-continuity data.

Specifically, the wearable-eye-movement-amount-continuity-data-acquisition section 24 calculates an eye movement amount Vn using the vertical direction potential Vv and the horizontal direction potential Vh calculated in Step S2, based on Expression 3 below. The eye movement amount Vn is data related to a magnitude of the eye potential.

(Expression 3)

$$Vn = (Vv^2 + Vh^2)^{1/2}$$

A time point where the eye movement amount Vn exceeds a predetermined threshold is a start of eye motion. A time after eye motion starts up to a time point where the eye movement amount Vn falls below the predetermined threshold is an eye motion zone. Note that the predetermined threshold is, for example, a maximum value calculated by Expression 3 from the measured eye potential when the user gazes forward. As used herein, the term "eye motion" means motion of the eyes relative to the head. That is, the eye motion means movement of the eyes relative to the head. The eye motion includes saccade (saccadic eye motion) in which, for example, an absolute value of an eye rotation angular velocity is a threshold $\alpha$ (for example, 30 deg/sec) or more and an absolute value of a motion amount obtained by integrating the rotation angular velocity is a threshold $\beta$ (for example, 3.5 deg) or more.

The maximum value of the eye movement amount Vn in each eye motion zone is acquired as an eye movement amount in the eye motion zone. Thus, the eye movement amount is continuously acquired. The wearable-eye-movement-amount-continuity data acquired by continuously acquiring the eye movement amount by the wearable-eye-movement-amount-continuity-data-acquisition section 24 is stored in the storage 21. Thereafter, a flow illustrated in FIG. 5 is terminated (END).

The wearable-electrooculography-continuity data and the wearable-eye-movement-amount-continuity data that have been acquired in the above described manner are calibrated by the correction section 60, as described above.

Calibration of the wearable-eye-movement-amount-continuity data by the correction section 60 will be described below.

(Calibration While Spectacle-Type-Wearable Device Is Worn)

A face shape differs for each user, and therefore, a positional relation among the left nose electrode 3 provided in the left nose pad 16L, the right nose electrode 4 provided in the right nose pad 16R, and the eyes in the spectacle-type-wearable device 1 differs for each user in some cases. Therefore, when the user wears the spectacle-type-wearable device 1, there is a deviation between actual eye motion and eye motion measured by the spectacle-type-wearable device 1 in some cases.

In order to correct an error between a measured value and a true value while the spectacle-type-wearable device 1 is worn, calibration of the wearable-eye-movement-amount-continuity data is performed in accordance with steps described below.

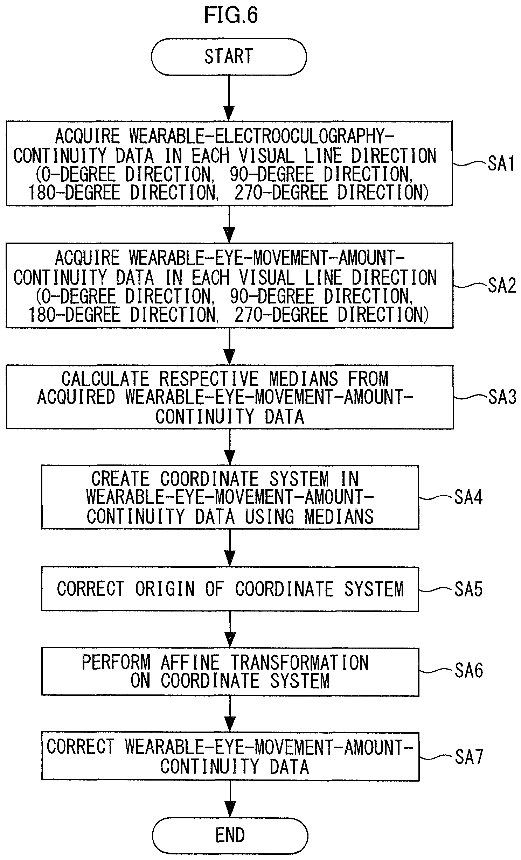

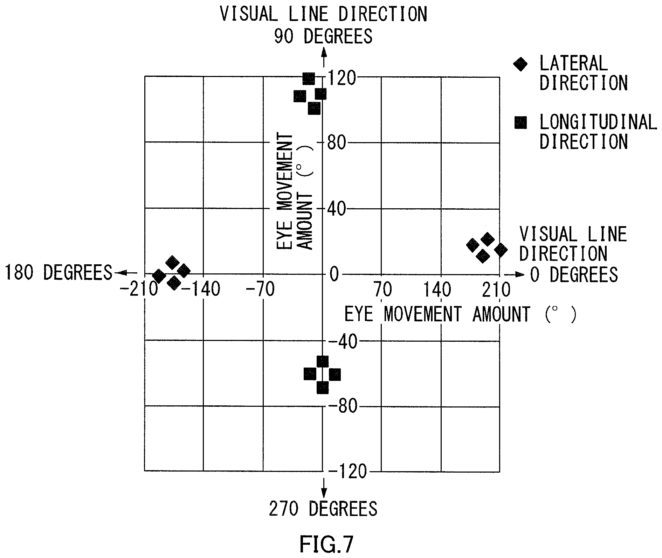

FIG. 6 is a flowchart illustrating an example of process steps for correcting the wearable-eye-movement-amount-continuity data while the spectacle-type-wearable device 1 is worn. FIG. 7 to FIG. 11 are explanatory charts each schematically illustrating an example of how the wearable-eye-movement-amount-continuity data is corrected.

When the spectacle-type-wearable device 1 is worn, calibration is performed in order to eliminate the error depending on a face shape, a wearing state, or the like of the user. Specifically, the continuous wearable-eye-movement-amount-continuity data is corrected by the correction section 60.

To perform the calibration, the eye potentials when the user moves a visual line upward, downward, leftward, and rightward in a state in which the user wears the spectacle-type-wearable device 1 are continuously measured by the electrooculography measuring device 30, and thereby, it is necessary to acquire the wearable-electrooculography-continuity data. Therefore, the eye potential at each of angles of 0 degrees, 90 degrees, 180 degrees, and 270 degrees when the user sequentially moves the visual line in directions of 0 degrees, 90 degrees, 180 degrees, and 270 degrees in a state in which the user wears the spectacle-type-wearable device 1 is continuously measured by the electrooculography measuring device 30. The above described angles are herein angles from 0 degrees in a counter-clock direction where a three o'clock direction on a clock as viewed from the user is 0 degrees.

That is, the user sequentially moves the visual line from a right side to an upper side, a left side, and a lower side in this order. In accordance with this visual line movement, the left eye potential VL and the right eye potential VR are continuously measured by the electrooculography measuring device 30. Measured data of this measurement is input to the wearable-device-compatible-electrooculography-data-processing device 20. The wearable-device-compatible-electrooculography-data-processing device 20 performs a calibration operation using the above described measured data.

First, in Step SA1 of the flowchart illustrated in FIG. 6, the wearable-electrooculography-continuity-data-acquisition section 23 acquires the wearable-electrooculography-continuity data in each visual line direction (a 0-degree direction, a 90-degree direction, a 180-degree direction, a 270-degree direction).

Subsequently, in Step SA2, the wearable-eye-movement-amount-continuity-data-acquisition section 24 acquires the wearable-eye-movement-amount-continuity data in each visual line direction (the 0-degree direction, the 90-degree direction, the 180-degree direction, the 270-degree direction), based on the wearable-electrooculography-continuity data in each visual line direction (the 0-degree direction, the 90-degree direction, the 180-degree direction, the 270-degree direction). Each of the wearable-eye-movement-amount-continuity data acquired by the wearable-eye-movement-amount-continuity-data-acquisition section 24 is stored in the storage 21.

FIG. 7 is an explanatory chart illustrating an example of the wearable-eye-movement-amount-continuity data acquired by the wearable-eye-movement-amount-continuity-data-acquisition section 24 by a graph of the movement amount in each visual line direction. As illustrated in FIG. 7, the wearable-eye-movement-amount-continuity data are plotted intensively in each of the 0-degree direction, the 90-degree direction, the 180-degree direction, and the 270-degree direction. Note that, in FIG. 7 to FIG. 11, an origin and each visual line direction are an origin and each visual line direction of a coordinate system (which will be hereinafter referred to as a reference coordinate system) set in the wearable-device-compatible-electrooculography-data-processing device 20. In FIG. 7 to FIG. 11, black squares indicate the wearable-eye-movement-amount-continuity data when the visual line moves in an up-down (longitudinal) direction and black lozenges indicate the wearable-eye-movement-amount-continuity data when the visual line moves in the left-right direction (lateral) direction.

In Step SA3, the correction section 60 calculates a median in each visual line direction from the acquired wearable-eye-movement-amount-continuity data in each visual line direction (the 0-degree direction, the 90-degree direction, the 180-degree direction, the 270-degree direction).

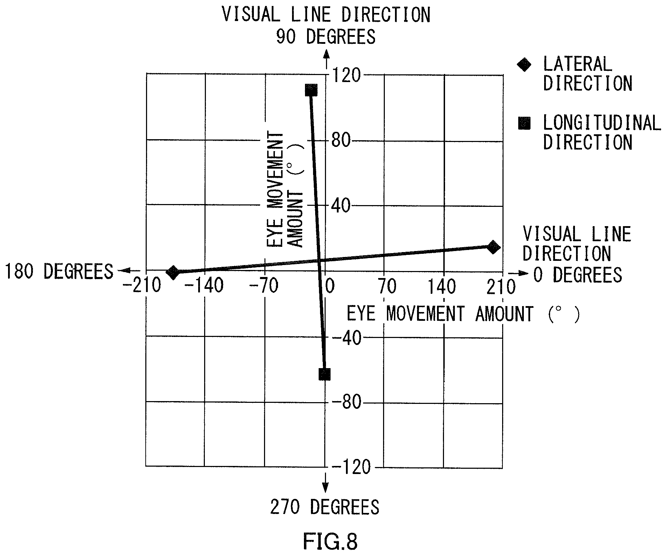

Subsequently, in Step SA4, the correction section 60 creates a coordinate system in the wearable-eye-movement-amount-continuity data using the medians calculated in Step SA3. FIG. 8 illustrates an example of the coordinate system in the wearable-eye-movement-amount-continuity data created by the correction section 60.

Specifically, the correction section 60 creates the coordinate system in the wearable-eye-movement-amount-continuity data by creating a vertical axis that connects two points (a median in the 90-degree direction and a median in the 270-degree direction) when the visual line moves in the up-down direction and a horizontal axis that connects two points (a median in the 0-degree direction and a median in the 180-degree direction) when a visual line moves in the left-right direction.

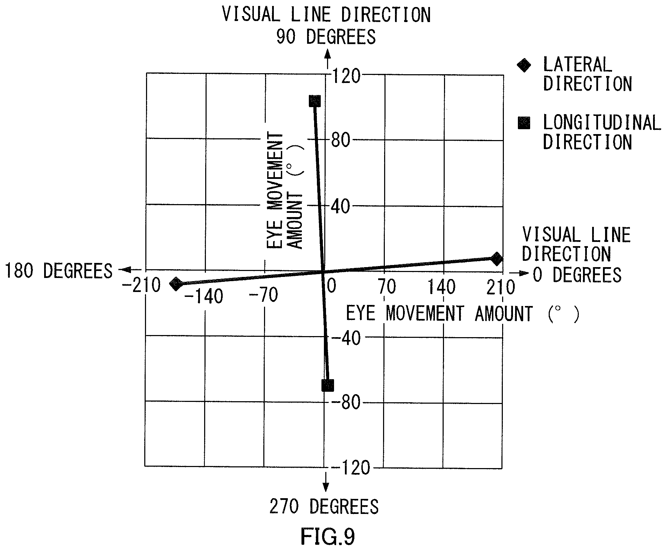

In subsequent Step SA5, as illustrated in FIG. 9, the correction section 60 corrects the coordinate system in the wearable-eye-movement-amount-continuity data such that an intersection of the vertical axis and the horizontal axis in the coordinate system created in Step SA4 is the origin of the reference coordinate system. Specifically, for the wearable-eye-movement-amount-continuity data, the correction section 60 corrects a difference between the intersection in the coordinate system in the wearable-eye-movement-amount-continuity data and the origin of the reference coordinate system. In this correction, a difference in the horizontal direction and a difference in the vertical direction are corrected such that the intersection and the origin of the reference coordinate system match each other.

In Step SA6, the correction section 60 performs affine transformation on the coordinate system in the wearable-eye-movement-amount-continuity data such that the vertical axis of the coordinate system in the wearable-eye-movement-amount-continuity data matches the vertical axis of the reference coordinate system and the horizontal axis of the coordinate system in the wearable-eye-movement-amount-continuity data matches the horizontal axis of the reference coordinate system. This affine transformation causes, by rotating the coordinate system in the wearable-eye-movement-amount-continuity data, the vertical axis of the coordinate system to match the vertical axis of the reference coordinate system and the horizontal axis of the coordinate system to match the horizontal axis of the reference coordinate system.

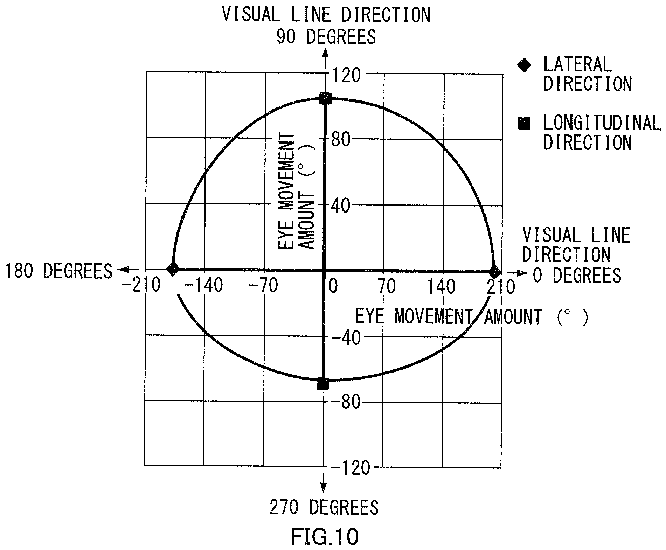

However, when only the above described affine transformation has been performed, as illustrated in FIG. 10, magnitudes of quadrants of the coordinate system in the wearable-eye-movement-amount-continuity data after the affine transformation are different from each other. That is, distortion occurs in each quadrant of the coordinate system.

Therefore, in Step SA7, the correction section 60 corrects the distortion in each quadrant of the coordinate system in the wearable-eye-movement-amount-continuity data after the affine transformation. That is, the correction section 60 corrects the median of the wearable-eye-movement-amount-continuity data in each of the visual line directions using the median of the wearable-eye-movement-amount-continuity data in the 0-degree visual line direction as a reference such that, in the quadrants of the coordinate system in the wearable-eye-movement-amount-continuity data after the affine transformation, arcs each of which connects corresponding ones of the medians of the wearable-eye-movement-amount-continuity data in the visual line directions are circular arcs having the same radius.

Specifically, the correction section 60 obtains a ratio between the median of the wearable-eye-movement-amount-continuity data in the 0-degree visual line direction and the median of the wearable-eye-movement-amount-continuity data in each of the other visual line directions (the 90-degree direction, the 180-degree direction, and the 270-degree direction). The correction section 60 uses, as a parameter used for correcting the wearable-eye-movement-amount-continuity data in each visual line direction, the ratio of the median of the wearable-eye-movement-amount-continuity data in each visual line direction to the median of the wearable-eye-movement-amount-continuity data in the 0-degree visual line direction, the ratio having been obtained in the above described manner.

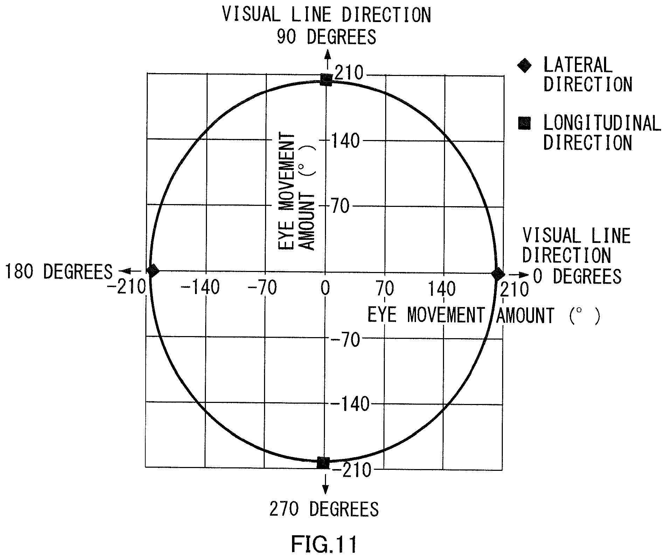

Specifically, for example, as illustrated in FIG. 10, when the medians of the wearable-eye-movement-amount-continuity data in the visual line directions are different from each other, the correction section 60 performs correction described below on the visual line of the wearable-eye-movement-amount-continuity data in each quadrant of the coordinate system after the above described calibration. The distortion in each quadrant is corrected, as illustrated in FIG. 11, by performing the correction described below.

<First Quadrant>

Data in the horizontal direction is not corrected, and data in the vertical direction is multiplied by a ratio between the median in the 0-degree visual line direction and the median in the 90-degree visual line direction.

<Second Quadrant>

Data in the horizontal direction is multiplied by a ratio between the median in the 0-degree visual line direction and the median in the 180-degree visual line direction. Data in the vertical direction is multiplied by the ratio between the median in the 0-degree visual line direction and the median in the 90-degree visual line direction.

<Third Quadrant>

Data in the horizontal direction is multiplied by the ratio between the median in the 0-degree visual line direction and the median in the 180-degree visual line direction.

Data in the vertical direction is multiplied by a ratio between the median in the 0-degree visual line direction and the median in the 270-degree visual line direction.

<Fourth Quadrant>

Data in the horizontal direction is not corrected, and data in the vertical direction is multiplied by the ratio between the median in the 0-degree visual line direction and the median in the 270-degree visual line direction.

As described above, the correction section 60 uses the obtained transformation parameter described above as a parameter used for correcting the wearable-eye-movement-amount-continuity data.

Thus, even when distortion occurs in the wearable-eye-movement-amount-continuity data while the spectacle-type-wearable device 1 is worn, the correction section 60 corrects the wearable-eye-movement-amount-continuity data using the parameter used for correction such that the wearable-eye-movement-amount-continuity data matches an actual eye movement amount. Note that the corrected wearable-eye-movement-amount-continuity data is stored in the storage 21.

Calibration of the wearable-eye-movement-amount-continuity data acquired by the wearable-eye-movement-amount-continuity-data-acquisition section 24 can be performed by the above described structure. Thus, when the eye potential of the user is measured using the spectacle-type-wearable device 1, highly accurate wearable-eye-movement-amount-continuity data can be obtained.

Note that, in this embodiment, calibration of the wearable-eye-movement-amount-continuity data by the correction section 60 has been described, but the correction section 60 may be configured to perform calibration on the wearable-electrooculography-continuity data in a similar manner.

The wearable-device-compatible-electrooculography-data-processing device 20 of the first embodiment is provided in the wearable device that user wears and can acquire eye movement amount continuity data of the user. Thus, the user wears the wearable device and can acquire the eye movement amount continuity data when the user exercises or performs an operation of a machine. The acquired wearable-eye-movement-amount-continuity data is related to an internal state when the user exercises or performs the operation of the machine and can be used in relative evaluation between users or the like for the internal state when the user exercises or performs the operation of the machine. Furthermore, the acquired visual line movement can be also used in evaluation and management of the internal state of the user in an operation or a work in a virtual space.

As used herein, the term "internal state" means an attention level (a state), an awake level (state), a concentration level (the degree of concentration), a margin, information processing performance, or the like. Furthermore, the internal state is not limited to a mental state and also means a physical state (a fatigue level).

For example, a state in which "the attention level is relatively high" is not a state in which an excessive attention is paid to a specific event but a state in which attentions can be paid to various events. A state in which "the attention level is relatively low" is a state in which an excessive attention is paid to a specific event (including a plurality of events) and attentions to other events are lost.

Note that, in embodiments of the present teaching, the arithmetic section 22 of the wearable-device-compatible-electrooculography-data-processing device 20 executes Steps S1 to S3 illustrated in FIG. 5 and Steps SA1 to SA7 illustrated in FIG. 6. The wearable-device-compatible-electrooculography-data-processing device 20 and a wearable-device-compatible-electrooculography-data-processing method in this embodiment can be realized by executing processing of Steps S1 to S3 and also executing processing of Steps SA1 to SA7 by the arithmetic section 22.

[Second Embodiment]

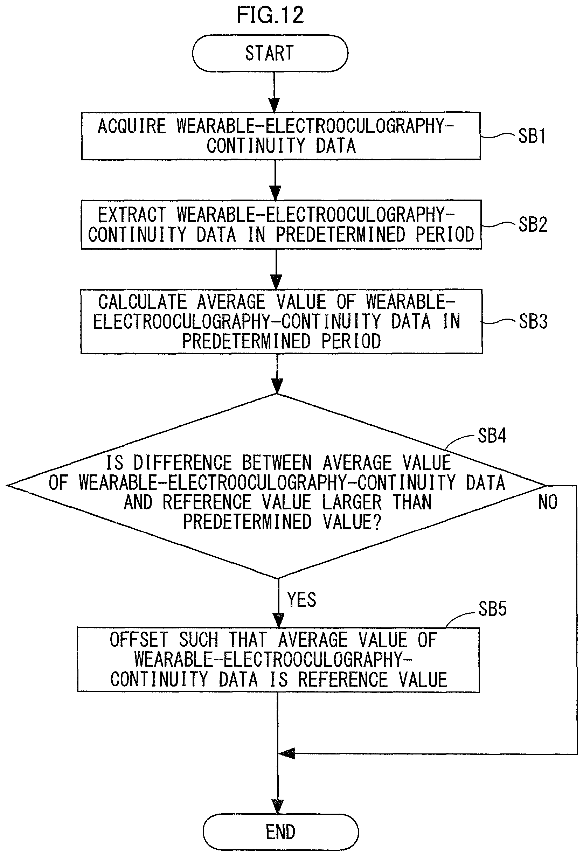

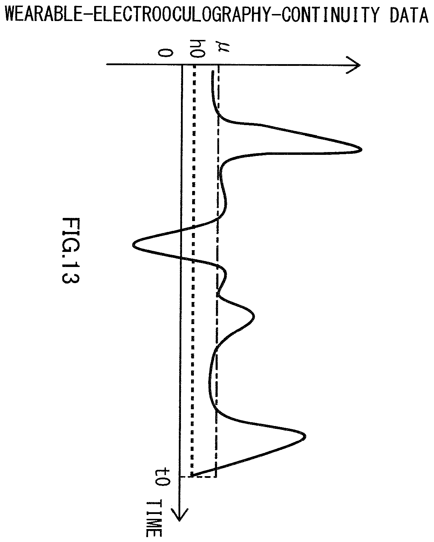

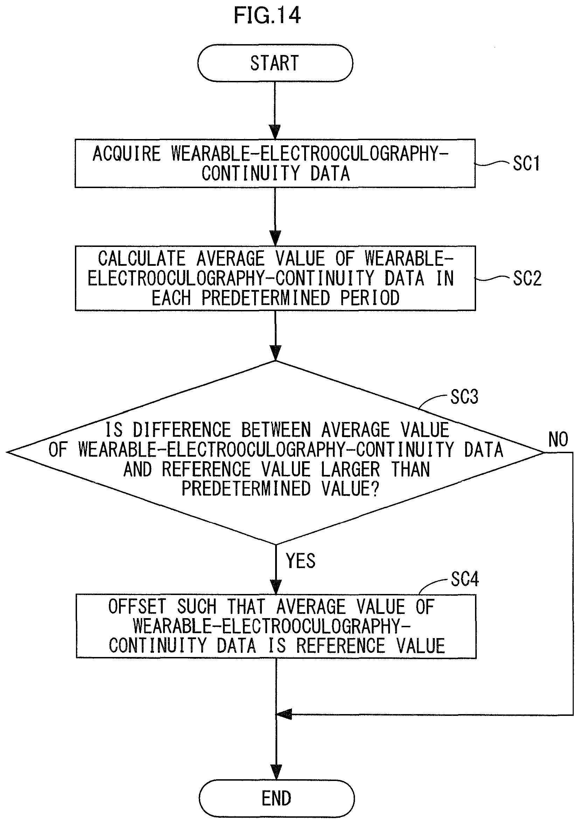

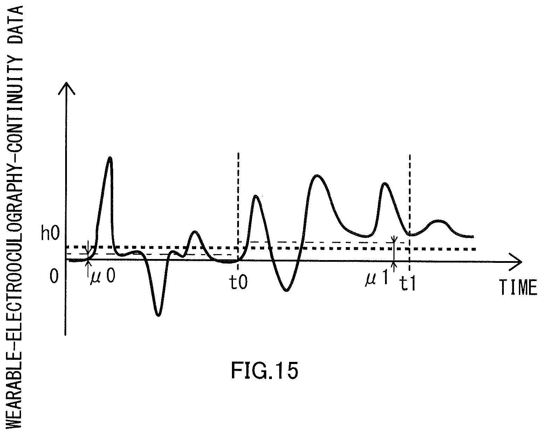

In FIG. 12 and FIG. 14, examples of process steps for correcting wearable-electrooculography-continuity data of a spectacle-type-wearable device according to a second embodiment of the present teaching are illustrated. FIG. 13 and FIG. 15 are explanatory charts schematically illustrating examples of how the wearable-electrooculography-continuity data is corrected. Note that a structure of the spectacle-type-wearable device of this embodiment is similar to the structure of the spectacle-type-wearable device 1 in the first embodiment, and therefore, detailed description of the structure will be omitted. In the following description, a similar component to a corresponding component in the first embodiment is denoted by the same reference sign as that of the corresponding component in the first embodiment, and the description for the component is omitted.

In this embodiment, a correction section 60 corrects wearable-electrooculography-continuity data using an eye potential measured by an electrooculography measuring device 30. Specifically, similar to the first embodiment, the correction section 60 performs calibration of the wearable-electrooculography-continuity data while a spectacle-type-wearable device 1 is worn.

The correction section 60 corrects the wearable-electrooculography-continuity data also when a position of each of electrodes (a reference electrode 2, a left nose electrode 3, and a right nose electrode 4) of the electrooculography measuring device 30 changes. That is, when the position of each of the electrodes (the reference electrode 2, the left nose electrode 3, and the right nose electrode 4) of the electrooculography measuring device 30 changes, the correction section 60 corrects the continuous wearable-electrooculography-continuity data or continuous wearable-eye-movement-amount-continuity data as appropriate. The correction section 60 reads the continuous wearable-electrooculography-continuity data or the continuous wearable-eye-movement-amount-continuity data from a storage 21, performs necessary correction processing, and stores corrected data in the storage 21.

(Calibration While Spectacle-Type-Wearable Device Is Worn)

FIG. 12 is a flowchart illustrating an example of process steps when the wearable-electrooculography-continuity data is calibrated while the spectacle-type-wearable device 1 is worn. FIG. 13 is an explanatory chart schematically illustrating an example of how the wearable-electrooculography-continuity data is corrected.

When wearable-electrooculography-continuity data that is acquired in a default position of the electrooculography measuring device 30 and wearable-electrooculography-continuity data actually acquired from the electrooculography measuring device 30 are different from each other while the spectacle-type-wearable device 1 is worn, the correction section 60 corrects the wearable-electrooculography-continuity data.

After the eye potential has been continuously measured by the electrooculography measuring device 30 for a predetermined time, the correction section 60 performs correction processing. Thus, in Step SB1, after the user wears the spectacle-type-wearable device 1, the electrooculography measuring device 30 continuously measures a left eye potential VL and a right eye potential VR, and a wearable-electrooculography-continuity-data-acquisition section 23 acquires the left eye potential VL and the right eye potential VR that have been measured and stores the left eye potential VL and the right eye potential VR in the storage 21.

In Step SB2, the correction section 60 extracts, from the wearable-electrooculography-continuity data stored in the storage 21, the wearable-electrooculography-continuity data during a predetermined period up to a predetermined time t0 since the spectacle-type-wearable device 1 was worn (a time 0). FIG. 13 illustrates an example of the wearable-electrooculography-continuity data during the predetermined period since the spectacle-type-wearable device 1 was worn. Note that the wearable-electrooculography-continuity data illustrated in FIG. 13 represents an example of the left eye potential VL. Although not particularly illustrated, the right eye potential VR is obtained in a similar manner to the manner in which the left eye potential VL is obtained.

In Step SB3, the correction section 60 obtains a time average value $\mu$ of the acquired wearable-electrooculography-continuity data in the predetermined period up to the predetermined time t0 since the wearable device was worn.

In Step SB4, the correction section 60 determines whether a different between the time average value $\mu$ and a reference value is larger than a predetermined value h0. The reference value is the default value set in advance for the spectacle-type-wearable device 1. For example, when the reference value is zero, the different between the time average value $\mu$ and the reference value is $\mu$. In this case, in Step SB4, the correction section 60 compares the time average value $\mu$ and the predetermined value h0 to each other. Even when a measurement error or the like occurs in the wearable-electrooculography-continuity data, the predetermined value h0 is set as a measurement result for the eye potential of the user in an allowable range.

In Step SB4, if it is determined that the time average value $\mu$ is larger than the predetermined value h0 (YES), the process proceeds to Step SB5. In Step SB5, the correction section 60 offsets the wearable-electrooculography-continuity data such that the time average value $\mu$ of the wearable-electrooculography-continuity data is zero that is the reference value. Thereafter, a correction operation illustrated in FIG. 12 is terminated (END).

On the other hand, in Step SB4, if it is determined that the time average value $\mu$ is the predetermined value h0 or less (NO), the correction section 60 does not correct the wearable-electrooculography-continuity data and terminates the correction operation (END).

As described above, whether the wearable-electrooculography-continuity data has changed with respect to the default reference value of the spectacle-type-wearable device 1 can be determined by using the time average value of the wearable-electrooculography-continuity data in the predetermined period up to the predetermined time since the spectacle-type-wearable device 1 was worn. That is, when the time average value of the wearable-electrooculography-continuity data has changed from the reference value, the wearable-electrooculography-continuity data can be corrected by offsetting the wearable-electrooculography-continuity data acquired after the predetermined time using the time average value. Accordingly, a wearable eye movement amount can be more accurately acquired.

Note that calibration of the wearable-electrooculography-continuity data may be performed using the method of the first embodiment.

(Correction When Position of Each Electrode of Electrooculography Measuring Device Changed)

When vibration or the like is applied to the spectacle-type-wearable device 1 in a state in which the spectacle-type-wearable device 1 is worn, a wearing position of the spectacle-type-wearable device 1 changes in some cases. When the wearing position changes as described above, a value of the wearable-electrooculography-continuity data acquired by the wearable-electrooculography-continuity-data-acquisition section 23 changes. Therefore, also when the wearing position of the spectacle-type-wearable device 1 changes, the correction section 60 performs correction processing. The correction processing of the correction section 60 while the spectacle-type-wearable device 1 is worn will be described.

FIG. 14 is a flowchart illustrating an example of process steps for correcting the wearable-electrooculography-continuity data when the position of each of the electrodes (the reference electrode 2, the left nose electrode 3, and the right nose electrode 4) of the electrooculography measuring device 30 while the spectacle-type-wearable device 1 is worn has changed. FIG. 15 is an explanatory chart schematically illustrating an example of how the correction section 60 corrects the wearable-electrooculography-continuity data.

When the wearable-electrooculography-continuity data changes due to change of the wearing position of the spectacle-type-wearable device 1 while the user wears the spectacle-type-wearable device 1, the correction section 60 corrects the wearable-electrooculography-continuity data.

In Step SC1, while the user wears the spectacle-type-wearable device 1, the electrooculography measuring device 30 continuously measures the left eye potential VL and the right eye potential VR. The wearable-electrooculography-continuity-data-acquisition section 23 acquires, as the wearable-electrooculography-continuity data, the left eye potential VL and the right eye potential VR that have been measured by the electrooculography measuring device 30 and stores the left eye potential VL and the right eye potential VR in the storage 21.

In Step SC2, the correction section 60 reads the wearable-electrooculography-continuity data in each predetermined period from the wearable-electrooculography-continuity-data stored in the storage 21 and calculates the time average value $\mu$ in each predetermined period. FIG. 15 illustrates an example of change of the wearable-electrooculography-continuity data while the user wears the spectacle-type-wearable device 1. Note that the example of the change of the wearable-electrooculography-continuity data illustrated in FIG. 15 is an example of change of the left eye potential VL. Although not particularly illustrated, for the right eye potential VR, similar data to that in FIG. 15 can be obtained by measuring the right eye potential VR by the electrooculography measuring device 30 in a similar manner.

In Step SC3, the correction section 60 determines whether the difference between the time average value $\mu$ and the reference value is larger than the predetermined value h0. The reference value is the default value set in advance for the spectacle-type-wearable device 1. Even when a measurement error or the like occurs in the wearable-electrooculography-continuity data, the predetermined value h0 is set as a measurement result for the eye potential of the user in an allowable range. For example, in the example of FIG. 15, a time average value $\mu 0$ of the wearable-electrooculography-continuity data in a period from the time 0 to a time t0 is substantially equal to a reference value (0 in the example of FIG. 15). In this case, the correction section 60 determines that the wearing position of the spectacle-type-wearable device 1 has not changed, and does not perform the correction processing.

In the example illustrated in FIG. 15, a difference between a time average value $\mu 1$ of the wearable-electrooculography-continuity data and a reference value in a period from the time t0 to the time t1 is larger than the predetermined value h0.

In this case, the difference between the time average value $\mu 1$ and the reference value ($\mu 1$ when the reference value is 0) is larger than the predetermined value h0. In Step SC3, if it is determined that the difference between the time average value $\mu 1$ and the reference value is larger than the predetermined value h0 (YES), the process proceeds to Step SC4. In Step SC4, the correction section 60 offsets the wearable-electrooculography-continuity data such that the time average value $\mu 1$ of the wearable-electrooculography-continuity data is zero that is the reference value. Thereafter, the correction section 60 terminates the correction operation.

As described above, whether the wearing position of the spectacle-type-wearable device 1 has changed can be detected by continuously acquiring the average value of the wearable-electrooculography-continuity data while the spectacle-type-wearable device 1 is worn. If the difference between the average value and the reference value is larger than the predetermined value h0, the correction section 60 can correct the wearable-electrooculography-continuity data while the spectacle-type-wearable device 1 is worn by offsetting the wearable-electrooculography-continuity data. Accordingly, the wearable-electrooculography-continuity data can be more accurately acquired.

Note that, in this embodiment, a case in which the correction section 60 corrects the wearable-electrooculography-continuity data in accordance with change of the wearing position of the spectacle-type-wearable device 1 has been described, but the correction section 60 may be configured to perform correction on the wearable-eye-movement-amount-continuity data in accordance with change of the wearing position of the spectacle-type-wearable device 1 in a similar manner.

[Third Embodiment]

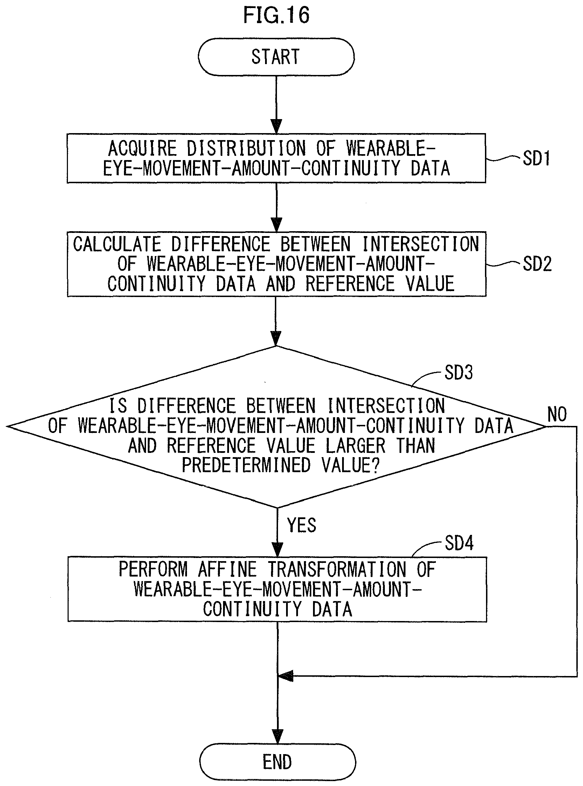

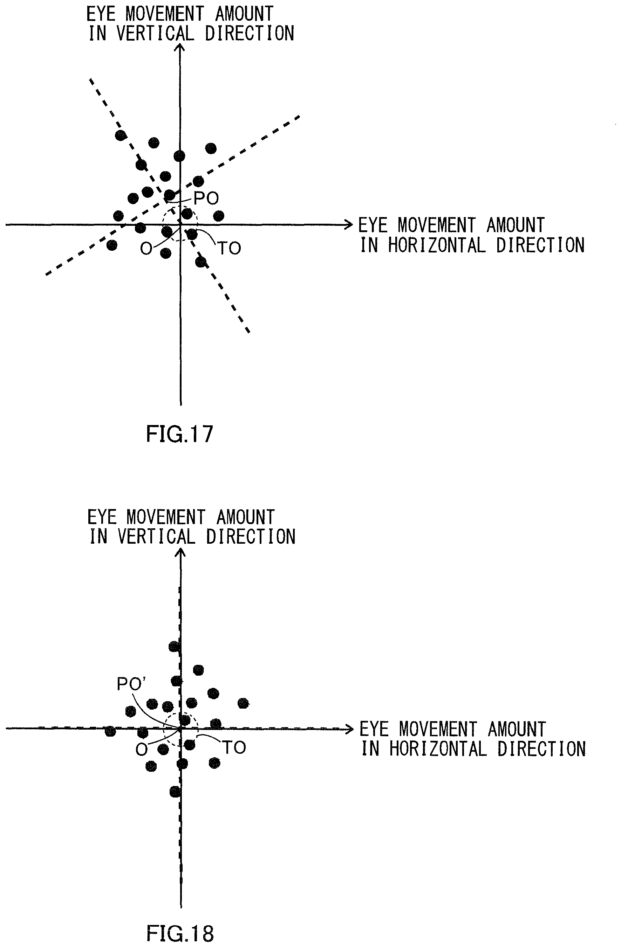

In FIG. 16, an example of process steps for correcting wearable-eye-movement-amount-continuity data of a spectacle-type-wearable device according to a third embodiment of the present teaching is illustrated. FIG. 17 and FIG. 18 are explanatory charts schematically illustrating examples of how the wearable-eye-movement-amount-continuity data is corrected. Note that a structure of the spectacle-type-wearable device of this embodiment is similar to the structure of the spectacle-type-wearable device 1 in the first embodiment, and therefore, detailed description of the structure will be omitted. In the following description, a similar component to a corresponding component in the first embodiment is denoted by the same reference sign as that of the corresponding component in the first embodiment, and the description for the component is omitted.

In this embodiment, a correction section 60 corrects the wearable-eye-movement-amount-continuity data when the wearable-eye-movement-amount-continuity data has changed due to change of a wearing position of the spectacle-type-wearable device 1 while a user wears the spectacle-type-wearable device 1.

An electrooculography measuring device 30 continuously measures a left eye potential VL and a right eye potential VR while the user wears the spectacle-type-wearable device 1. A wearable-electrooculography-continuity-data-acquisition section 23 acquires the measured data as wearable-electrooculography-continuity data and stores the wearable-electrooculography-continuity data in the storage 21. A wearable-eye-movement-amount-continuity-data-acquisition section 24 acquires wearable-eye-movement-amount-continuity data, based on the wearable-electrooculography-continuity data, and stores the acquired wearable-eye-movement-amount-continuity data in the storage 21.

The wearable-eye-movement-amount-continuity data is calculated as data of an eye movement amount in the left-right direction and the up-down direction by the wearable-eye-movement-amount-continuity-data-acquisition section 24. Therefore, information indicating in which quadrant in a coordinate system the wearable-eye-movement-amount-continuity data is located is associated with the wearable-eye-movement-amount-continuity data in accordance with the data of the eye movement amount in the left-right direction and the up-down direction, and the wearable-eye-movement-amount-continuity data is stored in the storage 21.

When the correction section 60 performs correction of the wearable-eye-movement-amount-continuity data while the spectacle-type-wearable device 1 is worn, the correction section 60 performs correction processing in accordance with the flowchart illustrated in FIG. 16.

First, in Step SD1, the correction section 60 reads wearable-eye-movement-amount-continuity data stored in the storage 21 and obtains a distribution of the wearable-eye-movement-amount-continuity data acquired while the spectacle-type-wearable device 1 is worn. The correction section 60 can calculate not only a distribution of the wearable-eye-movement-amount-continuity data but also calculate a horizontal axis and a vertical axis of a coordinate system in the wearable-eye-movement-amount-continuity data from the obtained distribution, as illustrated in FIG. 17. The correction section 60 also calculates an intersection P0 of the horizontal axis and the vertical axis that have been calculated.

Subsequently, in Step SD2, a difference between the calculated intersection P0 of the horizontal axis and the vertical axis in the coordinate system in the wearable-eye-movement-amount-continuity data and a reference value is obtained. For example, the reference value is an origin of a reference coordinate system set in the wearable-device-compatible-electrooculography-data-processing device 20.

In Step SD3, the correction section 60 determines whether the difference between the intersection P0 of the horizontal axis and the vertical axis in the coordinate system in the wearable-eye-movement-amount-continuity data and the reference value is larger than a predetermined value T0.

In Step SD3, if the correction section 60 determines that the intersection P0 of the horizontal axis and the vertical axis in the coordinate system in the wearable-eye-movement-amount-continuity data is the predetermined value T0 or less (NO), there is only small change, and therefore, the correction section 60 does not perform correction of the wearable-eye-movement-amount-continuity data and terminates the operation (END).

On the other hand, in Step SD3, if the correction section 60 determines that the intersection P0 of the horizontal axis and the vertical axis in the coordinate system in the wearable-eye-movement-amount-continuity data is larger than the predetermined value T0 (YES), the process proceeds to Step SD4.

In Step SD4, the correction section 60 performs correction in which the intersection P0 of the horizontal axis and the vertical axis in the coordinate system in the wearable-eye-movement-amount-continuity data is caused to match the reference value, correction in which the horizontal axis of the coordinate system is caused to match the horizontal axis of the reference coordinate system, and correction in which the vertical axis of the coordinate system is caused to match the vertical axis of the reference coordinate system.

Specifically, the correction section 60 performs affine transformation of the wearable-eye-movement-amount-continuity data. Thus, as illustrated in FIG. 18, through the affine transformation, the intersection P0 of the horizontal axis and the vertical axis in the coordinate system in the wearable-eye-movement-amount-continuity data is transformed into P0' to match the reference value, the horizontal axis of the coordinate system is caused to match the horizontal axis of the reference coordinate system, and the vertical axis of the coordinate system is caused to match the vertical axis of the reference coordinate system.

As described above, while the user wears the spectacle-type-wearable device 1, the wearable-eye-movement-amount-continuity-data-acquisition section 24 continuously acquires wearable-eye-movement-amount-continuity data, and thereby, a distribution of the wearable-eye-movement-amount-continuity data is obtained.

Thus, the correction section 60 can determine whether the difference between the intersection P0 of the horizontal axis and the vertical axis in the coordinate system in the wearable-eye-movement-amount-continuity data and the reference value has been increased. If the difference between the intersection P0 of the horizontal axis and the vertical axis in the coordinate system in the wearable-eye-movement-amount-continuity data and the reference value is larger than the predetermined value T0, the correction section 60 can correct wearable-eye-movement-amount-continuity data that is acquired while the user wears the spectacle-type-wearable device 1 by performing affine transformation of the wearable-eye-movement-amount-continuity data.

Accordingly, even when the wearable-eye-movement-amount-continuity data has changed due to change of the wearing position of the spectacle-type-wearable device 1, highly accurate wearable-eye-movement-amount-continuity data can be achieved by correcting wearable-eye-movement-amount-continuity data.

Note that, in this embodiment, correction of wearable-eye-movement-amount-continuity data by the correction section 60 has been described, but the correction section 60 may be configured to correct wearable-electrooculography-continuity data in a similar manner.

Similar to the first embodiment, the wearable-device-compatible-electrooculography-data-processing device of the second or third embodiment is provided in the wearable device that the user wears, and eye movement amount continuity data of the user can be acquired. Thus, the user wears the wearable device and can acquire the eye movement amount continuity data when the user exercises or performs an operation of a machine. The acquired wearable-eye-movement-amount-continuity data is related to an internal state when the user exercises or performs the operation of the machine and can be used in relative evaluation between users or the like for the internal state when the user exercises or performs the operation of the machine. Furthermore, the acquired visual line movement can be also used in evaluation and management of the internal state of the user in an operation and a work in a virtual space.

[Fourth Embodiment]

A person detects a visual object that is to be gazed at next by using information acquired from inside of the effective visual field and performs visual line movement by eye motion and head motion on the detected visual object. When the visual object is located in the effective visual field, the eye motion precedes the head motion. On the other hand, when the visual object is located outside the effective visual field, the head motion precedes the eye motion. As described above, depending on whether the visual object is located in the effective visual field, an action (which will be hereinafter referred to as a viewing action) of viewing the visual object by the person differs. Note that the viewing action includes at least one of motion of eyes (eye motion) and motion of a head (head motion). As used herein, the term "eye motion" means motion of the eyes relative to the head. That is, the eye motion means the movement of the eyes relative to the head. The eye motion includes saccade (saccadic eye motion) in which, for example, an absolute value of an eye rotation angular velocity is a threshold $\alpha$ (for example, 30 deg/sec) or more and an absolute value of a motion amount obtained by integrating the rotation angular velocity is a threshold $\beta$ (for example, 3.5 deg) or more. The head motion is motion of the head when the head moves or rotates in absolute coordinates in the up-down direction and the left-right direction. That is, the head motion includes motion of the head when the head moves or rotates relative to a body, motion of the head when the head and the body move or rotate together, and motion of the head when the head and the body separately move or rotate.

Incidentally, an area of an effective visual field of a person changes in accordance with an internal state of the person. As used herein, the term "internal state" means an attention level (state), an awake level (state), a concentration level (the degree of concentration), a margin, information processing performance, or the like.

Furthermore, the internal state is not limited to a mental state and also means a physical state (a fatigue level).

Specifically, the effective visual field of the person tends to be narrower as the internal state of the person becomes poorer. Therefore, the effective visual field of the person is detected, and thereby, the internal state of the person can be understood.

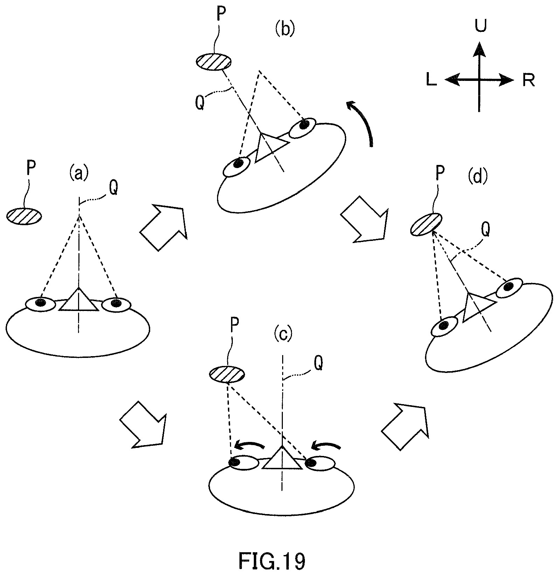

FIG 19 schematically illustrates an example of a viewing action. The person performs a viewing action so as to view a visual object P on a central line between left and right eyes in the left-right direction. Therefore, as illustrated in FIG. 19(a), when the visual object P is off a centerline Q (which will be hereinafter merely referred to as a centerline between the left and right eyes) located at an equal distance from each of the left eye and the right eye in the left-right direction, as illustrated in FIG. 19(d), the person views the visual object P on the centerline Q between the left and right eyes in the left-right direction by performing a viewing action illustrated in FIG. 19(b) or FIG. 19(c).

Note that, in the example of FIG. 19, at least either the eyes or the head of the person move or moves in an order of FIG. 19(a), FIG. 19(b), and FIG. 19(d) or in an order of FIG. 19(a), FIG. 19(c), and FIG. 19(d) over time. Also, in FIG. 19, the visual object P, not in a cross-sectional view, is indicated by oblique lines for use in describing the corresponding viewing action.

As illustrated in FIG. 19(a), when the visual object P is off the centerline Q between the left and right eyes in the left-right direction, the person moves the eyes and the head to view the visual object P on the centerline Q between the left and right eyes.

FIG. 19(b) schematically illustrates a state in which head motion occurs first. In this case, in the left-right direction, movement of the head is started such that the visual object P is located on the centerline Q between the left and right eyes. Thereafter, the person starts moving the left and right eyes and views the visual object P (FIG. 19(d)). As illustrated in FIG. 19(b), when the person views the visual object P with the left and right eyes, the person starts the head motion first in some cases. As described above, a viewing action in which a start of the head motion precedes a start of the eye motion in time is referred to as a "head preceding type viewing action" or merely as a "head preceding type."

FIG. 19(c) schematically illustrates a state in which eye motion occurs first. In this case, the person starts eye motion such that the visual object P is located on the centerline Q between the left and right eyes in the left-right direction. Thereafter, the person starts head motion (FIG. 19(d)). As illustrated in FIG. 19(c), when the person views the visual object P with the left and right eyes, the person starts the eye motion first in some cases. As described above, a viewing action in which a start of eye motion precedes a start of head motion in time will be referred to as an "eye preceding type viewing action" or merely as an "eye preceding type."

As descried above, in a case in which the visual object P is off the centerline between the left and right eyes in the left-right direction, when the person views the visual object P, there are the head preceding type viewing action and the eye preceding type viewing action as viewing actions.

Note that the person easily performs the eye preceding type viewing action when the visual object is located in the effective visual field and easily performs the head preceding type viewing action when the visual object is not located in the effective visual field. Therefore, as the effective visual field becomes narrower, a visual object is less likely to be located in the effective visual field, and therefore, the head preceding type viewing action tends to occur in many cases.

As described above, when the person performs the viewing action to view the visual object on the central line between the left and right eyes, whether the viewing action is a head preceding type viewing action or an eye preceding type viewing action is influenced by the effective visual field of the person. Then, as described above, the effective visual field of the person is influenced by the internal state of the person. Therefore, the effective visual field related to the internal state of the person can be relatively evaluated by analyzing the head preceding type viewing action and the eye preceding type viewing action in the viewing action.

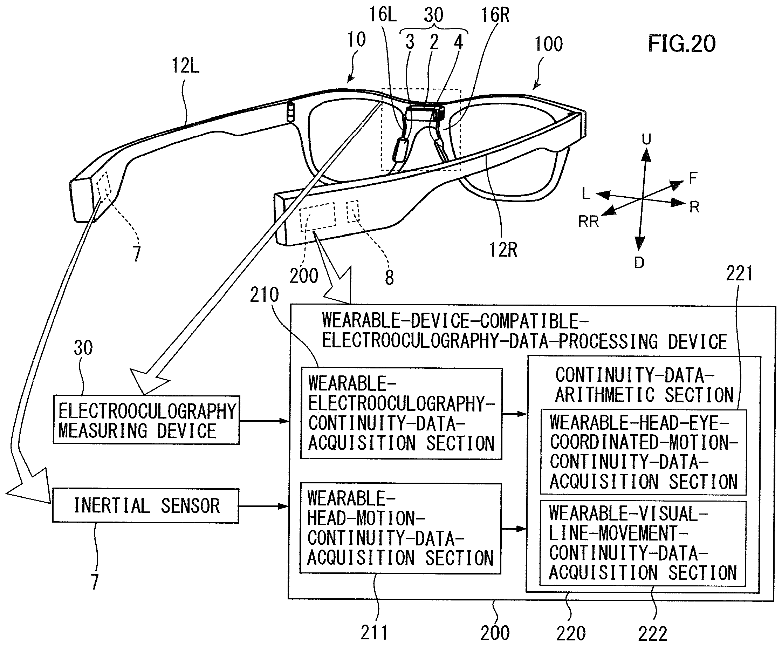

In FIG. 20, an outline structure of a spectacle-type-wearable device 100 according to a fourth embodiment is illustrated. The spectacle-type-wearable device 100 of this embodiment detects a viewing action by detecting a visual line movement by eye motion

(eye movement) and head motion. The spectacle-type-wearable device 100 includes, in addition to the components of the spectacle-type-wearable device 1 of the first embodiment, a head-motion-measuring device 7 that detects head motion. The spectacle-type-wearable device 100 includes a wearable-device-compatible-electrooculography-data-processing device 200, instead of the wearable-device-compatible-electrooculography-data-processing device 20 in the spectacle-type-wearable device 1 of the first embodiment.

The structure of the spectacle-type-wearable device 100 of this embodiment is similar to the structure of the spectacle-type-wearable device 1 in the first embodiment, except the above described structure, and therefore, a detailed description of the structure will be omitted. In the following description, a similar component to a corresponding component in the first embodiment is denoted by the same reference sign as that of the corresponding component in the first embodiment, and the description for the component is omitted.

The head-motion-measuring device 7 is, for example, incorporated in a left temple 12L. The head-motion-measuring device 7 is, for example, an inertial sensor. Specifically, the head-motion-measuring device 7 may be a six-axis gyro sensor, and may be formed of only an acceleration sensor or only an angular velocity sensor. The head-motion-measuring device 7 may be configured to estimate head motion from imaged data obtained by an image sensor.

The head-motion-measuring device 7 outputs head motion data resulting from head motion of a user. The head motion data is time series data related to an angular velocity and an angle of the head (information related to an angular velocity and an angle of the head at each time).

The output head motion data is given to the wearable-device-compatible-electrooculography-data-processing device 200. The wearable-device-compatible-electrooculography-data-processing device 200 includes a wearable-electrooculography-continuity-data-acquisition section 210, a wearable-head-motion-continuity-data-acquisition section 211, and a continuity-data-arithmetic section 220.

An electrooculography measuring device 30 continuously outputs an eye potential. The wearable-electrooculography-continuity-data-acquisition section 210 continuously acquires, as wearable-electrooculography-continuity data, the eye potential continuously output from the electrooculography measuring device 30. The acquired wearable-electrooculography-continuity data is output to the continuity-data-arithmetic section 220.

The wearable-head-motion-continuity-data-acquisition section 211 continuously acquires a sensor output continuously output from the head-motion-measuring device 7. The wearable-head-motion-continuity-data-acquisition section 211 calculates, based on the sensor output that has been input, wearable-head-motion-continuity data and outputs the calculated wearable-head-motion-continuity data to the continuity-data-arithmetic section 220.

The continuity-data-arithmetic section 220 includes a wearable-head-eye-coordinated-motion-continuity-data-acquisition section 221 and a wearable-visual-line-movement-amount-continuity-data-acquisition section 222.

The wearable-head-eye-coordinated-motion-continuity-data-acquisition section 221 calculates, from wearable-electrooculography-continuity data and wearable-head-motion-continuity data, continuous wearable-head-eye-coordinated-motion-continuity data related to a coordinated motion of eye movement (eye motion) and head motion while the spectacle-type-wearable device 100 is used. Furthermore, the wearable-head-eye-coordinated-motion-continuity-data-acquisition section 221 calculates wearable-eye-movement-amount-continuity data from the wearable-electrooculography-continuity data.

The wearable-visual-line-movement-amount-continuity-data-acquisition section 222 calculates, based on the wearable-eye-movement-amount-continuity data and the wearable-head-eye-coordinated-motion-continuity data, wearable-visual-line-movement-amount-continuity data that corresponds to predetermined wearable-head-eye-coordinated-motion-continuity data while the spectacle-type-wearable device 100 is used.

The wearable-device-compatible-electrooculography-data-processing device 200 transmits the wearable-visual-line-movement-amount-continuity data that corresponds to the predetermined wearable-head-eye-coordinated-motion-continuity data from a communication section (not illustrated) to an external processing device (not illustrated). The external processing device (not illustrated) is, for example, configured to determine an internal state of the user, based on the transmitted wearable-visual-line-movement-amount-continuity data that corresponds to the predetermined wearable-head-eye-coordinated-motion-continuity data, and perform various notifications to the user.

The wearable-device-compatible-electrooculography-data-processing device 200 acquires and outputs a visual line movement related value related to the visual line movement of the user, based on each of measurement results of the electrooculography measuring device 30 and the head-motion-measuring device 7.

The wearable-electrooculography-continuity-data-acquisition section 210 acquires the eye potential of the user continuously measured by the electrooculography measuring device 30 as wearable-electrooculography-continuity data, as described above. The wearable-head-motion-continuity-data-acquisition section 211 acquires wearable-head-motion-continuity data of the user from continuous measured data obtained from the head-motion-measuring device 7.

The continuity-data-arithmetic section 220 calculates the visual line movement related value that is a value related to the visual line movement of the user, based on the wearable-electrooculography-continuity data and the wearable-head-motion-continuity data of the user acquired by the wearable-electrooculography-continuity-data-acquisition section 210 and the wearable-head-motion-continuity-data-acquisition section 211.

Specifically, the continuity-data-arithmetic section 220 continuously calculates an eye movement amount from the wearable-electrooculography-continuity data, continuously calculates a head movement amount of the head motion data corresponding to the eye movement data, and continuously obtains a sum of the eye movement amount and the head movement amount. The continuity-data-arithmetic section 220 specifies a start timing of head motion and a start timing of eye movement of the user and determines which one of the head motion or the eye movement precedes, based on the specified timings.

Herein, the visual-line-movement amount of the user is represented by the total of the eye movement amount and the head movement amount of the user. That is, in the fourth embodiment, the sum of the eye movement amount and the head movement amount is the visual-line-movement amount of the user. Therefore, the continuity-data-arithmetic section 220 continuously obtains the visual-line-movement amount of the user.

More specifically, the continuity-data-arithmetic section 220 includes the wearable-head-eye-coordinated-motion-continuity-data-acquisition section 221 and the wearable-visual-line-movement-amount-continuity-data-acquisition section 222.

The wearable-head-eye-coordinated-motion-continuity-data-acquisition section 221 has a function of calculating an eye movement amount, a function of specifying eye movement, a function of specifying head motion, and a function of associating motions. The wearable-visual-line-movement-amount-continuity-data-acquisition section 222 has a function of calculating a visual-line-movement amount and a function of determining preceding motion.

The function of the wearable-head-eye-coordinated-motion-continuity-data-acquisition section 221 to specify eye movement calculates an eye movement amount from wearable-electrooculography-continuity data obtained by the wearable-electrooculography-continuity-data-acquisition section 210.

The function of the wearable-head-eye-coordinated-motion-continuity-data-acquisition section 221 to specify eye movement specifies a zone of the eye motion and specifies a start timing of the eye movement from the eye movement amount of the user.

The function of the wearable-head-eye-coordinated-motion-continuity-data-acquisition section 221 to specify head motion specifies a zone of head motion and specifies a start timing of the head motion from the head motion data of the user acquired by the wearable-head-motion-continuity-data-acquisition section 211.

The function of the wearable-head-eye-coordinated-motion-continuity-data-acquisition section 221 to associate motions associates calculated eye movement and calculated head motion with each other at a timing at which the eye movement and the head motion are specified.

The function of the wearable-visual-line-movement-amount-continuity-data-acquisition section 222 to calculate the visual-line-movement amount calculates, as a visual-line-movement amount, a total of the motion amounts (an integrated value of the rotation angular velocity) in zones in which respective motions are specified in the eye movement and the head motion associated with each other.

The function of the wearable-visual-line-movement-amount-continuity-data-acquisition section 222 to determine preceding motion determines preceding motion of the eye movement and the head motion associated with each other.

(Operation of Wearable-Device-Compatible-Electrooculography-Data-Processing Device 200)

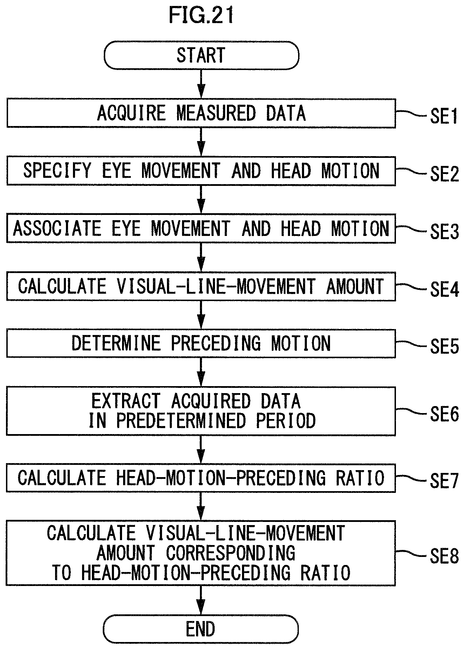

Next, an operation of the wearable-device-compatible-electrooculography-data-processing device 200 will be described. In FIG. 21, a flowchart of the operation of the wearable-device-compatible-electrooculography-data-processing device 200 is illustrated.

When a flow illustrated in FIG. 21 starts, in Step SE1, the wearable-electrooculography-continuity-data-acquisition section 210 and the wearable-head-motion-continuity-data-acquisition section 211 acquire continuously measured data from each of the electrooculography measuring device 30 and the head-motion-measuring device 7.

Next, in Step SE2, the wearable-head-eye-coordinated-motion-continuity-data-acquisition section 221 calculates an eye movement amount from wearable-electrooculography-continuity data obtained by the wearable-electrooculography-continuity-data-acquisition section 210. The function of specifying eye movement specifies the eye movement and specifies a start timing of the eye movement, based on calculated eye movement data.

In Step SE2, the function of specifying head motion of the wearable-head-eye-coordinated-motion-continuity-data-acquisition section 221 specifies head motion and specifies a start timing of the head motion, based on head motion data acquired by the wearable-head-motion-continuity-data-acquisition section 211.

In subsequent Step SE3, the function of associating motions of the wearable-head-eye-coordinated-motion-continuity-data-acquisition section 221 associates the calculated eye movement and head motion with each other at a timing at which the eye movement and the head motion are specified.

In Step SE4, the function of calculating a visual-line-movement amount of the wearable-visual-line-movement-amount-continuity-data-acquisition section 222 calculates, as a visual-line-movement amount, a total of the motion amounts (an integrated value of the rotation angular velocity) in zones in which respective motions are specified in the eye movement and the head motion associated with each other.

In Step SE5, the function of determining preceding motion of the wearable-visual-line-movement-amount-continuity-data-acquisition section 222 determines preceding motion of the eye movement and the head motion associated with each other.

Specifically, when there is no motion specified to be the head motion in the zone in which the eye movement is specified, it is determined that the eye movement precedes. Even in a case in which there is motion specified to be the head motion in the zone in which the eye movement is specified, when the start of the eye movement is earlier than the start of the head motion, the function of determining preceding motion determines that the eye movement precedes.

On the other hand, in a case in which there is motion specified to be the eye movement in the zone in which the head motion is specified, when the start of the head motion is earlier than the start of the eye motion, the function of determining preceding motion determines that the head motion precedes.

A determination result (preceding motion information) of determination of preceding motion is linked to the visual-line-movement amount calculated by the wearable-visual-line-movement-amount-continuity-data-acquisition section 222.

In Step SE6, acquired data in a predetermined period is extracted from data in which the preceding motion information and the visual-line-movement amount are linked to each other.

In Step SE7, a ratio of data in which the head motion precedes, that is, a head-motion-preceding ratio, in the predetermined number of extracted data is calculated.

In Step SE8, after the visual-line-movement amount corresponding to the head-motion-preceding ratio is calculated, the operation is terminated.

Based on the foregoing, in this embodiment, a visual-line-movement amount corresponding to a head-motion-preceding ratio can be obtained. That is, for example, a visual-line-movement amount when the head-motion-preceding ratio is a predetermined value can be obtained. As already described above, the head-motion-preceding ratio is influenced by an effective visual field of a person. Therefore, the visual-line-movement amount when the head-motion-preceding ratio is the predetermined value is a parameter related to the effective visual field of the person. Accordingly, an internal state of the person can be estimated using the visual-line-movement amount.

The user can wear the wearable device of the fourth embodiment and can obtain the visual-line-movement amount corresponding to the acquired head-motion-preceding ratio. The visual-line-movement amount corresponding to the head-motion-preceding ratio can be provided as data related to the effective visual field when the internal state of the user is estimated. For example, when the user exercises or performs an operation of a machine, the parameter related to the effective visual field is acquired by the wearable device of the fourth embodiment, and thereby, the acquired parameter related to the effective visual field can be used in determination of the internal state of the user or the like when the user exercises or performs the operation of the machine. The acquired parameter related to the effective visual field can be also used in relative evaluation between users or the like for the internal state when the user exercises or performs the operation of the machine. Furthermore, the acquired visual-line-movement amount can be also used in evaluation and management of the internal state of the user in an operation or a work in a virtual space.

(Other Embodiments)

Embodiments of the present teaching have been described above, but the above-described embodiments are merely illustrative examples of a preferred embodiment of the present teaching. Therefore, the present teaching is not limited to the above-described embodiments and the above-described embodiments can be appropriately modified and implemented without departing from the gist of the teaching.

In each of the above described embodiments, the wearable-electrooculography-continuity-data-acquisition section 23 acquires wearable-electrooculography-continuity data by an arithmetic operation, based on a measured value of the electrooculography measuring device 30. However, the wearable-electrooculography-continuity-data-acquisition section 23 may be configured to acquire the wearable-electrooculography-continuity data using a table.

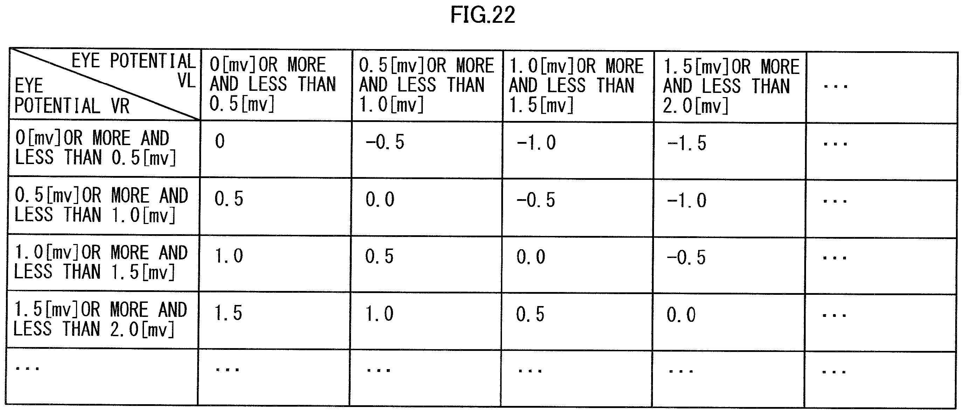

In FIG. 22, an example of a table used for acquiring the wearable-electrooculography-continuity data is illustrated. The table is a data in which a left eye potential VL and a right eye potential VR are associated with wearable-electrooculography-continuity data in the vertical direction and is stored in the storage 21. Thus, the wearable-electrooculography-continuity-data-acquisition section 23 can acquire the wearable-electrooculography-continuity data in the vertical direction from the left eye potential VL and the right eye potential VR measured by the electrooculography measuring device 30.

Note that, similarly, for electrooculography continuity data in the horizontal direction, the left eye potential VL and the right eye potential VR may be associated with wearable-electrooculography-continuity data in the horizontal direction and may be stored in the storage 21 as table data. Thus, the wearable-electrooculography-continuity-data-acquisition section can acquire the wearable-electrooculography-continuity data in the horizontal direction from the right eye potential VR and the left eye potential VL measured by the electrooculography measuring device 30.

In each of the above described embodiments, the wearable-device-compatible-electrooculography-data-processing device 20, 200 is provided in the right temple 12R of the spectacle-type-wearable device 1. However, the wearable-device-compatible-electrooculography-data-processing device may be provided in some other position than the right temple 12R of the spectacle-type-wearable device 1. In the spectacle-type-wearable device 1, a position in which the wearable-device-compatible-electrooculography-data-processing device is provided is determined by a relationship between sizes and weights of the spectacle-type-wearable device 1 and the wearable-device-compatible-electrooculography-data-processing device.

In each of the above described embodiments, the left nose electrode 3 and the right nose electrode 4 are provided in the left nose pad 16L and the right nose pad 16R, respectively. The reference electrode 2 is provided in the bridge 13. However, the left nose electrode 3, the right nose electrode 4, and the reference electrode 2 may be provided in any positions in the spectacle-type-wearable device 1 in which the eye potentials of the left eye and the right eye of the user wearing the spectacle-type-wearable device 1 can be measured.

In each of the above described embodiments, the spectacle-type-wearable device 1 measures the eye potentials of the left eye and the right eye of the user. However, the spectacle-type-wearable device may be configured to measure only an eye potential in one position and may be configured to measure eye potentials in three or more positions. Note that, even in a case in which the number of eye potentials to be measured is some other number than two, an electrode may be provided in any position in which the eye potential of the user can be measured. For example, a pair of electrodes may be provided above and below one of the right eye or the left eye, and electrodes may be provided at a right end of the eyelid of the right eye or at a left end of the eyelid of the left eye.

In each of the above described embodiments, the correction section 60 corrects wearable-electrooculography-continuity data using only the wearable-electrooculography-continuity data. The correction section 60 also corrects the wearable-electrooculography-continuity data using only wearable-eye-movement-amount-continuity data. However, the correction section 60 may be configured to correct the wearable-electrooculography-continuity data using both of the wearable-electrooculography-continuity data and the wearable-eye-movement-amount-continuity data, and may be configured to correct the wearable-eye-movement-amount-continuity data using both of the wearable-electrooculography-continuity data and the wearable-eye-movement-amount-continuity data. The correction section 60 may be configured to correct both of the wearable-electrooculography-continuity data and the wearable-eye-movement-amount-continuity data using both of the wearable-electrooculography-continuity data and the wearable-eye-movement-amount-continuity data.

Furthermore, the correction section 60 may be configured to correct the wearable-eye-movement-amount-continuity data using the wearable-electrooculography-continuity data, and may be configured to correct the wearable-electrooculography-continuity data using the wearable-eye-movement-amount-continuity data.

In each of the above described embodiments, the wearable-electrooculography-continuity-data-acquisition section 23 acquires, as the wearable-electrooculography-continuity data, the vertical direction potential and the horizontal direction potential calculated from left and right eye potentials. However, the wearable-eye-movement-amount-continuity-data-acquisition section 24 may be configured to calculate, after the wearable-electrooculography-continuity-data-acquisition section 23 acquires the left and right eye potentials, the vertical direction potential and the horizontal direction potential.

In each of the above described embodiments, the storage 21 includes the data storage 21a and the program storage 21b. However, the data storage 21a and the program storage 21b may be formed of separate storage devices. Note that the program storage 21b may be provided in an external information processing device.

In each of the above described embodiments, the wearable-device-compatible-electrooculography-data-processing device 20, 200 is provided in the spectacle-type-wearable device 1. However, the wearable-device-compatible-electrooculography-data-processing device 20, 200 may be provided in a wearable device that a user can wear and can measure an eye potential. For example, the wearable-device-compatible-electrooculography-data-processing device 20, 200 may be provided in a sunglass-type-wearable device, a goggle-type-wearable device, a head-mount-display-type-wearable device, or the like.

In each of the above described embodiments, as an example, the wearable-device-compatible-electrooculography-data-processing device 20 includes the storage 21 that performs various types of processing, based on a program stored in the program storage 21b. However, the wearable-device-compatible-electrooculography-data-processing device may be configured to perform various types of processing using a structure of a hardware, such as a circuit or the like.

INDUSTRIAL APPLICABILITY

The present teaching is applicable to a wearable-device-compatible-electrooculography-data-processing device that acquires, using an electrooculography measuring device provided in a wearable device that a user can wear and can continuously measure an eye potential of the user, continuous wearable-eye-movement-amount-continuity data related to an eye movement amount of the user while the wearable device is used.

REFERENCE SIGNS LIST

| | |
|---|---|
| 1, 100 | Spectacle-type-wearable device |
| 2 | Reference electrode |
| 3 | Left nose electrode |
| 4 | Right nose electrode |
| 6L | Left lens |
| 6R | Right lens |
| 7 | Head-motion-measuring device |
| 8 | Power source |
| 10 | Frame |
| 20, 200 | Wearable-device-compatible-electrooculography-data-processing device |
| 23 | Wearable-electrooculography-continuity-data-acquisition section |
| 24 | Wearable-eye-movement-amount-continuity-data-acquisition section |
| 30 | Electrooculography measuring device |
| 40 | Communication section |
| 60 | Correction section |
| 221 | Wearable-head-eye-coordinated-motion-continuity-data-acquisition section |

222 Wearable-visual-line-movement-amount-
continuity-data-acquisition section

**Claims**

1. A wearable-device-compatible-electrooculography-
data-processing device (20, 200),

   wherein the wearable-device-compatible-electrooculography-data-processing device (20,
   200) is configured to continuously acquire wearable-electrooculography-continuity data from
   an electrooculography measuring device (30)
   provided in a wearable device (1, 100) that a
   user wears, the electrooculography measuring
   device (30) being configured to continuously
   measure an eye potential of the user to provide
   the wearable-electrooculography-continuity data based on the continuously measured eye potential,
   wherein the wearable-device-compatible-electrooculography-data-processing device (20,
   200) is configured to acquire, based on the wear-
   able-electrooculography-continuity data contin-
   uously acquired while the wearable device (1,
   100) is used, continuous wearable-eye-movement-amount-continuity data related to an eye
   movement amount of the user while the wearable device (1, 100) is used, and
   **characterised in that** the wearable-device-
   compatible-electrooculography-data-processing device (20, 200) is configured to detect a
   change of a position of the electrooculography
   measuring device (30) while the wearable device (1, 100) is worn by use of the wearable-
   electrooculography-continuity data or the wearable-eye-movement-amount-continuity data.

2. The wearable-device-compatible-electrooculography-data-processing device (20, 200) according to
   claim 1,
   wherein the wearable-device-compatible-electrooculography-data-processing device (20, 200) is configured to correct at least one of the wearable-electrooculography-continuity data or the wearable-eye-
   movement-amount-continuity data based on at least
   one of the wearable-electrooculography-continuity
   data or the wearable-eye-movement-amount-continuity data.

3. The wearable-device-compatible-electrooculography-data-processing device (20, 200) according to
   claim 1 or 2,

   wherein the electrooculography measuring device (30) is configured to continuously measure
   eye potentials of the user at a plurality of positions, and
wherein for each of the plurality of positions a
plurality of wearable-electrooculography-continuity data are continuously acquired based on
the eye potentials at the plurality of positions,
the eye potentials having been continuously
measured by the electrooculography measuring
device (30).

4. The wearable-device-compatible-electrooculography-data-processing device (20, 200) according to
   any one of claims 1 to 3,
   wherein the wearable-eye-movement-amount-continuity data is data related to a magnitude of the eye
   potential.

5. The wearable-device-compatible-electrooculography-data-processing device (20, 200) according to
   any one of claims 1 to 4,

   wherein the wearable-device-compatible-electrooculography-data-processing device (20,
   200) is configured to continuously acquire wearable-head-motion-continuity data of the user
   from a head-motion-measuring device (7) provided in the wearable device (1, 100),
   wherein the wearable-device-compatible-electrooculography-data-processing device (20,
   200) is configured to acquire wearable head
   movement amount continuity data from the continuously acquired wearable-head-motion-continuity data, and
   wherein the wearable-device-compatible-electrooculography-data-processing device (20,
   200) is configured to acquire wearable-visual-
   line-movement-amount-continuity data from the
   wearable-eye-movement-amount-continuity
   data and the wearable head movement amount
   continuity data.

6. The wearable-device-compatible-electrooculography-data-processing device (20, 200) according to
   claim 5,

   wherein the wearable-device-compatible-electrooculography-data-processing device (20,
   200) is configured to acquire, from the wearable-
   electrooculography-continuity data and the
   wearable-head-motion-continuity data, continuous wearable-head-eye-coordinated-motion-
   continuity data related to a coordinated motion
   of eye motion and head motion while the wearable device (1, 100) is used, and
   wherein the wearable-device-compatible-electrooculography-data-processing device (20,
   200) is configured to output the wearable-visual-
   line-movement-amount-continuity data that corresponds to the predetermined wearable-head-eye-coordinated-motion-continuity data.

7. The wearable-device-compatible-electrooculography-data-processing device (20, 200) according to any one of claims 1 to 6, comprising:

a wearable-electrooculography-continuity-data-acquisition section (23) configured to continuously acquire the wearable-electrooculography-continuity data from the electrooculography measuring device (30); and
a wearable-eye-movement-amount-continuity-data-acquisition section (24) configured to acquire, based on the wearable-electrooculography-continuity data acquired by the wearable-electrooculography-continuity-data-acquisition section (23), while the wearable device (1, 100) is used, the continuous wearable-eye-movement-amount-continuity data.

8. A spectacle-type-wearable device (1, 100) comprising:
the wearable-device-compatible-electrooculography-data-processing device (20, 200) according to any one of claims 1 to 7.

9. A wearable-device-compatible-electrooculography-data-processing method comprising:

continuously acquiring wearable-electrooculography-continuity data from an electrooculography measuring device (30) provided in a wearable device (1, 100) that a user wears and that is configured to continuously measure an eye potential of the user to provide the wearable-electrooculography-continuity data based on the continuously measured eye potential;
acquiring, based on the wearable-electrooculography-continuity data continuously acquired, while the wearable device (1, 100) is used, continuous wearable-eye-movement-amount-continuity data related to an eye movement amount of the user while the wearable device (1, 100) is used; and **characterised in**
detecting a change of a position of the electrooculography measuring device (30) while the wearable device (1, 100) is worn by use of the wearable-electrooculography-continuity data or the wearable-eye-movement-amount-continuity data.

10. The wearable-device-compatible-electrooculography-data-processing method according to claim 9, wherein at least one of the wearable-electrooculography-continuity data or the wearable-eye-movement-amount-continuity data is corrected based on at least one of the wearable-electrooculography-continuity data or the wearable-eye-movement-amount-continuity data.

11. The wearable-device-compatible-electrooculography-data-processing method according to claim 9 or 10,

wherein the electrooculography measuring device (30) continuously measures eye potentials of the user at a plurality of positions, and
wherein for each of the plurality of positions a plurality of wearable-electrooculography-continuity data are continuously acquired based on the eye potentials at the plurality of positions, the eye potentials having been continuously measured by the electrooculography measuring device (30).

12. The wearable-device-compatible-electrooculography-data-processing method according to any one of claims 9 to 11,
wherein the wearable-eye-movement-amount-continuity data is data related to a magnitude of the eye potential.

13. The wearable-device-compatible-electrooculography-data-processing method according to any one of claims 9 to 12, further comprising:

continuously acquiring wearable-head-motion-continuity data of the user from a head-motion-measuring device (7) provided in the wearable device (1, 100),
acquiring wearable head movement amount continuity data from the continuously acquired wearable-head-motion-continuity data, and
acquiring wearable-visual-line-movement-amount-continuity data from the wearable-eye-movement-amount-continuity data and the wearable head movement amount continuity data.

14. The wearable-device-compatible-electrooculography-data-processing method according to claim 13, further comprising:

acquiring, from the wearable-electrooculography-continuity data and the wearable-head-motion-continuity data, continuous wearable-head-eye-coordinated-motion-continuity data related to a coordinated motion of eye motion and head motion while the wearable device (1, 100) is used, and
outputting the wearable-visual-line-movement-amount-continuity data that corresponds to the predetermined wearable-head-eye-coordinated-motion-continuity data.

**Patentansprüche**

1. Eine mit einer Wearable-Vorrichtung kompatible Elektrookulographie-Datenverarbeitungsvorrichtung (20, 200),

   wobei die mit einer Wearable-Vorrichtung kompatible Elektrookulographie-Datenverarbeitungsvorrichtung (20, 200) dazu konfiguriert ist, Wearable-Elektrookulographie-Kontinuitätsdaten von einer Elektrookulographie-Messvorrichtung (30), die in einer Wearable-Vorrichtung (1, 100) vorgesehen ist, die ein Benutzer trägt, kontinuierlich zu erfassen, wobei die Elektrookulographie-Messvorrichtung (30) dazu konfiguriert ist, ein Augenpotential des Benutzers kontinuierlich zu messen, um die Wearable-Elektrookulographie-Kontinuitätsdaten auf Basis des kontinuierlich gemessenen Augenpotentials bereitzustellen,
   wobei die mit einer Wearable-Vorrichtung kompatible Elektrookulographie-Datenverarbeitungsvorrichtung (20, 200) dazu konfiguriert ist, auf Basis der Wearable-Elektrookulographie-Kontinuitätsdaten, die während der Verwendung der Wearable-Vorrichtung (1, 100) kontinuierlich erfasst werden, kontinuierliche Wearable-Augenbewegungsbetrag-Kontinuitätsdaten in Bezug auf einen Augenbewegungsbetrag des Benutzers während der Verwendung der Wearable-Vorrichtung (1, 100) zu erfassen, und **dadurch gekennzeichnet, dass**
   die mit einer Wearable-Vorrichtung kompatible Elektrookulographie-Datenverarbeitungsvorrichtung (20, 200) dazu konfiguriert ist, eine Änderung einer Position der Elektrookulographie-Messvorrichtung (30), während die Wearable-Vorrichtung (1, 100) getragen wird, mit Hilfe der Wearable-Elektrookulographie-Kontinuitätsdaten oder der Wearable-Augenbewegungsbetrag-Kontinuitätsdaten zu detektieren.

2. Die mit einer Wearable-Vorrichtung kompatible Elektrookulographie-Datenverarbeitungsvorrichtung (20, 200) gemäß Anspruch 1,
   wobei die mit einer Wearable-Vorrichtung kompatible Elektrookulographie-Datenverarbeitungsvorrichtung (20, 200) dazu konfiguriert ist, zumindest entweder die Wearable-Elektrookulographie-Kontinuitätsdaten oder die Wearable-Augenbewegungsbetrag-Kontinuitätsdaten auf Basis von zumindest entweder den Wearable-Elektrookulographie-Kontinuitätsdaten oder den Wearable-Augenbewegungsbetrag-Kontinuitätsdaten zu korrigieren.

3. Die mit einer Wearable-Vorrichtung kompatible Elektrookulographie-Datenverarbeitungsvorrichtung (20, 200) gemäß Anspruch 1 oder 2,
   wobei die Elektrookulographie-Messvorrichtung (30) dazu konfiguriert ist, Augenpotentiale des Benutzers an einer Mehrzahl von Positionen kontinuierlich zu messen, und
   wobei für jede der Mehrzahl von Positionen eine Mehrzahl von Wearable-Elektrookulographie-Kontinuitätsdaten auf Basis der Augenpotentiale an der Mehrzahl von Positionen kontinuierlich erfasst wird, wobei die Augenpotentiale durch die Elektrookulographie-Messvorrichtung (30) kontinuierlich gemessen wurden.

4. Die mit einer Wearable-Vorrichtung kompatible Elektrookulographie-Datenverarbeitungsvorrichtung (20, 200) gemäß einem der Ansprüche 1 bis 3, wobei die Wearable- Augenbewegungsbetrag-Kontinuitätsdaten Daten sind, die sich auf eine Größe des Augenpotentials beziehen.

5. Die mit einer Wearable-Vorrichtung kompatible Elektrookulographie-Datenverarbeitungsvorrichtung (20, 200) gemäß einem der Ansprüche 1 bis 4,

   wobei die mit einer Wearable-Vorrichtung kompatible Elektrookulographie-Datenverarbeitungsvorrichtung (20, 200) dazu konfiguriert ist, Wearable-Kopfbewegung-Kontinuitätsdaten des Benutzers von einer Kopfbewegungs-Messvorrichtung (7), die in der Wearable-Vorrichtung (1, 100) vorgesehen ist, kontinuierlich zu erfassen, wobei die mit einer Wearable-Vorrichtung kompatible Elektrookulographie-Datenverarbeitungsvorrichtung (20, 200) dazu konfiguriert ist, Wearable-Kopfbewegungsbetrag-Kontinuitätsdaten aus den kontinuierlich erfassten Wearable-Kopfbewegung-Kontinuitätsdaten zu erfassen, und
   wobei die mit einer Wearable-Vorrichtung kompatible Elektrookulographie-Datenverarbeitungsvorrichtung (20, 200) dazu konfiguriert ist, Wearable-Blicklinienbewegungsbetrag-Kontinuitätsdaten aus den Wearable-Augenbewegungsbetrag-Kontinuitätsdaten und den Wearable-Kopfbewegungsbetrag-Kontinuitätsdaten zu erfassen.

6. Die mit einer Wearable-Vorrichtung kompatible Elektrookulographie-Datenverarbeitungsvorrichtung (20, 200) gemäß Anspruch 5,

   wobei die mit einer Wearable-Vorrichtung kompatible Elektrookulographie-Datenverarbeitungsvorrichtung (20, 200) dazu konfiguriert ist, aus den Wearable-Elektrookulographie-Kontinuitätsdaten und den Wearable-Kopfbewegung-Kontinuitätsdaten kontinuierliche Wearable-Koordinierte-Kopf-Augen-Bewegung-Kontinuitätsdaten in Bezug auf eine koordinierte Bewegung von Augenbewegung und Kopfbewegung zu erfassen, während die Wearable-Vorrichtung (1, 100) verwendet wird, und wobei die mit der Wearable-Vorrichtung kompatible Elektrookulographie-Datenverarbeitungsvorrichtung (20, 200) dazu konfiguriert ist, die Wearable-Blicklinienbewegungsbetrag-Kontinuitätsdaten auszugeben, die den vorbestimmten Wearable-Koordinierte-Kopf-Augen-Bewegung-Kontinuitätsdaten entsprechen.

7. Die mit einer Wearable-Vorrichtung kompatible Elektrookulographie-Datenverarbeitungsvorrichtung (20, 200) gemäß einem der Ansprüche 1 bis 6, die folgende Merkmale aufweist:
einen Wearable-Elektrookulographie-Kontinuitätsdaten-Erfassungsabschnitt (23), der dazu konfiguriert ist, die Wearable-Elektrookulographie-Kontinuitätsdaten von der Elektrookulographie-Messvorrichtung (30) kontinuierlich zu erfassen; und einen Wearable-Augenbewegungsbetrag-Kontinuitätsdaten-Erfassungsabschnitt (24), der dazu konfiguriert ist, auf Basis der Wearable-Elektrookulographie-Kontinuitätsdaten, die durch den Wearable-Elektrookulographie-Kontinuitätsdaten-Erfassungsabschnitt (23) während der Verwendung der Wearable-Vorrichtung (1, 100) erfasst werden, die kontinuierlichen Wearable-Augenbewegungsbetrag-Kontinuitätsdaten zu erfassen.

8. Eine Wearable-Vorrichtung vom Brillentyp (1, 100), die folgendes Merkmal aufweist:
die mit einer Wearable-Vorrichtung kompatible Elektrookulographie-Datenverarbeitungsvorrichtung (2, 200) gemäß einem der Ansprüche 1 bis 7.

9. Ein mit einer Wearable-Vorrichtung kompatibles Elektrookulographie-Datenverarbeitungsverfahren, das folgende Schritte aufweist:

kontinuierliches Erfassen von Wearable-Elektrookulographie-Kontinuitätsdaten von einer Elektrookulographie-Messvorrichtung (30), die in einer Wearable-Vorrichtung (1, 100) vorgesehen ist, die ein Benutzer trägt und die dazu konfiguriert ist, ein Augenpotential des Benutzers kontinuierlich zu messen, um die Wearable-Elektrookulographie-Kontinuitätsdaten auf Basis des kontinuierlich gemessenen Augenpotentials bereitzustellen;
Erfassen, auf Basis der Wearable-Elektrookulographie-Kontinuitätsdaten, die während der Verwendung der Wearable-Vorrichtung (1, 100) kontinuierlich erfasst werden, von kontinuierlichen Wearable-Augenbewegungsbetrag-Kontinuitätsdaten in Bezug auf einen Augenbewegungsbetrag des Benutzers während der Verwendung der Wearable-Vorrichtung (1, 100);

und
**gekennzeichnet durch**
Detektieren einer Änderung einer Position der Elektrookulographie-Messvorrichtung (30), während die Wearable-Vorrichtung (1, 100) getragen wird, mit Hilfe der Wearable-Elektrookulographie-Kontinuitätsdaten oder der Wearable-Augenbewegungsbetrag-Kontinuitätsdaten.

10. Das mit einer Wearable-Vorrichtung kompatible Elektrookulographie-Datenverarbeitungsverfahren gemäß Anspruch 9,
wobei zumindest entweder die Wearable-Elektrookulographie-Kontinuitätsdaten oder die Wearable-Augenbewegungsbetrag-Kontinuitätsdaten auf Basis von zumindest entweder den Wearable-Elektrookulographie-Kontinuitätsdaten oder den Wearable-Augenbewegungsbetrag-Kontinuitätsdaten korrigiert werden.

11. Das mit einer Wearable-Vorrichtung kompatible Elektrookulographie-Datenverarbeitungsverfahren gemäß Anspruch 9 oder 10,

wobei die Elektrookulographie-Messvorrichtung (30) Augenpotentiale des Benutzers an einer Mehrzahl von Positionen kontinuierlich misst, und
wobei für jede der Mehrzahl von Positionen eine Mehrzahl von Wearable-Elektrookulographie-Kontinuitätsdaten auf Basis der Augenpotentiale an der Mehrzahl von Positionen kontinuierlich erfasst wird, wobei die Augenpotentiale durch die Elektrookulographie-Messvorrichtung (30) kontinuierlich gemessen wurden.

12. Das mit einer Wearable-Vorrichtung kompatible Elektrookulographie-Datenverarbeitungsverfahren gemäß einem der Ansprüche 9 bis 11,
wobei die Wearable-Augenbewegungsbetrag-Kontinuitätsdaten Daten sind, die sich auf eine Größe des Augenpotentials beziehen.

13. Das mit einer Wearable-Vorrichtung kompatible Elektrookulographie-Datenverarbeitungsverfahren gemäß einem der Ansprüche 9 bis 12, das ferner folgende Schritte aufweist:

kontinuierliches Erfassen von Wearable-Kopfbewegung-Kontinuitätsdaten des Benutzers von einer Kopfbewegung-Messvorrichtung (7), die in der Wearable-Vorrichtung (1, 100) vorgesehen ist,
Erfassen von Wearable-Kopfbewegungsbetrag-Kontinuitätsdaten aus den kontinuierlich erfassten Wearable-Kopfbewegung-Kontinuitätsdaten, und
Erfassen von Wearable-Blicklinienbewegungsbetrag-Kontinuitätsdaten aus den Wearable-Augenbewegungsbetrag-Kontinuitätsdaten und den Wearable-Kopfbewegungsbetrag-Kontinuitätsdaten.

14. Das mit einer Wearable-Vorrichtung kompatible Elektrookulographie-Datenverarbeitungsverfahren gemäß Anspruch 13, das ferner folgende Schritte aufweist:

Erfassen, aus den Wearable-Elektrookulographie-Kontinuitätsdaten und den Wearable-Kopfbewegung-Kontinuitätsdaten, von kontinuierlichen Wearable-Koordinierte-Kopf-Augen-Bewegung-Kontinuitätsdaten in Bezug auf eine koordinierte Bewegung von Augenbewegung und Kopfbewegung, während die Wearable-Vorrichtung (1, 100) verwendet wird, und Ausgeben der Wearable-Blicklinienbewegungsbetrag-Kontinuitätsdaten, die den vorbestimmten Wearable-Koordinierte-Kopf-Augen-Bewegung-Kontinuitätsdaten entsprechen.

## Revendications

1. Appareil de traitement de données d'électro-oculographie compatible avec un appareil portable (20, 200),

dans lequel le dispositif de traitement de données d'électro-oculographie compatible avec un dispositif portable (20, 200) est configuré pour acquérir en continu les données de continuité d'électro-oculographie portable à partir d'un dispositif de mesure d'électro-oculographie (30) prévu dans un dispositif portable (1, 100) que porte un utilisateur, le dispositif de mesure d'électro-oculographie (30) étant configuré pour mesurer en continu un potentiel des yeux de l'utilisateur pour fournir les données de continuité d'électro-oculographie portable sur base du potentiel des yeux mesuré en continu, dans lequel le dispositif de traitement de données d'électro-oculographie compatible avec un dispositif portable (20, 200) est configuré pour acquérir, sur base des données de continuité d'électro-oculographie portable acquises en continu tandis que le dispositif portable (1, 100) est utilisé, les données de continuité de quantité de mouvement des yeux portable continues relatives à une quantité de mouvement des yeux de l'utilisateur tandis que le dispositif portable (1, 100) est utilisé, et **caractérisé par le fait que** le dispositif de traitement de données d'électro-oculographie compatible avec un dispositif portable (20, 200) est configuré pour détecter un changement d'une position du dispositif de mesure d'électro-oculographie (30) tandis que le dispositif portable (1, 100) est porté à l'aide des données de continuité d'électro-oculographie portable ou des données de continuité de quantité de mouvement des yeux portable.

2. Dispositif de traitement de données d'électro-oculographie compatible avec un dispositif portable (20, 200) selon la revendication 1, dans lequel le dispositif de traitement de données d'électro-oculographie compatible avec un dispositif portable (20, 200) est configuré pour corriger au moins les unes parmi les données de continuité d'électro-oculographie portable ou les données de continuité de quantité de mouvement des yeux portable sur base d'au moins les unes parmi les données de continuité d'électro-oculographie portable ou les données de continuité de quantité de mouvement des yeux portable.

3. Dispositif de traitement de données d'électro-oculographie compatible avec un dispositif portable (20, 200) selon la revendication 1 ou 2,

dans lequel le dispositif de mesure d'électro-oculographie (30) est configuré pour mesurer en continu les potentiels des yeux de l'utilisateur à une pluralité de positions, et dans lequel, pour chacune de la pluralité de positions, une pluralité de données de continuité d'électro-oculographie portable sont acquises en continu sur base des potentiels des yeux à la pluralité de positions, les potentiels des yeux ayant été mesurés en continu par le dispositif de mesure d'électro-oculographie (30).

4. Dispositif de traitement de données d'électro-oculographie compatible avec un dispositif portable (20, 200) selon l'une quelconque des revendications 1 à 3, dans lequel les données de continuité de quantité de mouvement des yeux portable sont des données relatives à une amplitude du potentiel des yeux.

5. Dispositif de traitement de données d'électro-oculographie compatible avec un dispositif portable (20, 200) selon l'une quelconque des revendications 1 à 4,

dans lequel le dispositif de traitement de données d'électro-oculographie compatible avec un dispositif portable (20, 200) est configuré pour acquérir en continu les données de continuité de mouvement de la tête portable de l'utilisateur à partir d'un dispositif de mesure de mouvement de la tête (7) prévu dans le dispositif portable (1, 100),

dans lequel le dispositif de traitement de données d'électro-oculographie compatible avec un dispositif portable (20, 200) est configuré pour acquérir les données de continuité de quantité de mouvement de la tête portable à partir des données de continuité de mouvement de la tête portable acquises en continu, et

dans lequel le dispositif de traitement de données d'électro-oculographie compatible avec un dispositif portable (20, 200) est configuré pour acquérir les données de continuité de quantité de mouvement de ligne de vision portable à partir des données de continuité de quantité de mouvement des yeux portable et des données de continuité de quantité de mouvement de la tête portable.

6. Dispositif de traitement de données d'électro-oculographie compatible avec un dispositif portable (20, 200) selon la revendication 5,

dans lequel le dispositif de traitement de données d'électro-oculographie compatible avec un dispositif portable (20, 200) est configuré pour acquérir, à partir des données de continuité d'électro-oculographie portable et des données de continuité de mouvement de la tête portable, les données de continuité de mouvement coordonné de la tête et des yeux portable relatives à un mouvement coordonné du mouvement des yeux et du mouvement de la tête tandis que le dispositif portable (1, 100) est utilisé, et

dans lequel le dispositif de traitement de données d'électro-oculographie compatible avec un dispositif portable (20, 200) est configuré pour sortir les données de continuité de quantité de mouvement de ligne de vision portable qui correspondent aux données de continuité de mouvement coordonné de la tête et des yeux portable prédéterminées.

7. Dispositif de traitement de données d'électro-oculographie compatible avec un dispositif portable (20, 200) selon l'une quelconque des revendications 1 à 6, comprenant:

un segment d'acquisition de données de continuité d'électro-oculographie portable (23) configuré pour acquérir en continu les données de continuité d'électro-oculographie portable à partir du dispositif de mesure d'électro-oculographie (30); et

un segment d'acquisition de données de continuité de quantité de mouvement des yeux portable (24) configuré pour acquérir, sur base des données de continuité d'électro-oculographie portable acquises par le segment d'acquisition de données de continuité d'électro-oculographie portable (23) tandis que le dispositif portable (1, 100) est utilisé, les données de continuité de quantité de mouvement des yeux portable continues.

8. Dispositif portable de type lunettes (1, 100) comprenant:
le dispositif de traitement de données d'électro-oculographie compatible avec un dispositif portable (20, 200) selon l'une quelconque des revendications 1 à 7.

9. Procédé de traitement de données d'électro-oculographie compatible avec un dispositif portable comprenant le fait de:

acquérir en continu les données de continuité d'électro-oculographie portable à partir d'un dispositif de mesure d'électro-oculographie (30) prévu dans un dispositif portable (1, 100) que porte un utilisateur et qui est configuré pour mesurer en continu un potentiel des yeux de l'utilisateur pour fournir les données de continuité d'électro-oculographie portable sur base du potentiel des yeux mesuré en continu;

acquérir, sur base des données de continuité d'électro-oculographie portable acquises en continu tandis que le dispositif portable (1, 100) est utilisé, les données de continuité de quantité de mouvement des yeux portable continues relatives à une quantité de mouvement des yeux de l'utilisateur tandis que le dispositif portable (1, 100) est utilisé; et

**caractérisé par** le fait de

détecter un changement d'une position du dispositif de mesure d'électro-oculographie (30) tandis que le dispositif portable (1, 100) est porté à l'aide des données de continuité d'électro-oculographie portable ou des données de continuité de quantité de mouvement des yeux portable.

10. Procédé de traitement de données d'électro-oculographie compatible avec un dispositif portable selon la revendication 9,
dans lequel au moins les unes parmi les données de continuité d'électro-oculographie portable ou les données de continuité de quantité de mouvement des yeux portable sont corrigées sur base d'au moins les unes parmi les données de continuité d'électro-oculographie portable ou les données de continuité de quantité de mouvement des yeux portable.

11. Procédé de traitement de données d'électro-oculographie compatible avec un dispositif portable selon la revendication 9 ou 10,

dans lequel le dispositif de mesure d'électro-oculographie (30) mesure en continu les potentiels des yeux de l'utilisateur à une pluralité de positions, et

dans lequel, pour chacune de la pluralité de positions, une pluralité de données de continuité d'électro-oculographie portable sont acquises en continu sur base des potentiels des yeux à la pluralité de positions, les potentiels des yeux ayant été mesurés en continu par le dispositif de mesure d'électro-oculographie (30).

12. Procédé de traitement de données d'électro-oculographie compatible avec un dispositif portable selon l'une quelconque des revendications 9 à 11, dans lequel les données de continuité de quantité de mouvement des yeux portable sont des données relatives à une amplitude du potentiel des yeux.

13. Procédé de traitement de données d'électro-oculographie compatible avec un dispositif portable selon l'une quelconque des revendications 9 à 12, comprenant par ailleurs le fait de:

acquérir en continu les données de continuité de mouvement de la tête de l'utilisateur portable à partir d'un dispositif de mesure de mouvement de la tête (7) prévu dans le dispositif portable (1, 100),

acquérir les données de continuité de quantité de mouvement de la tête portable à partir des données de continuité de mouvement de la tête portable acquises en continu, et

acquérir les données de continuité de quantité de mouvement de la ligne de vision portable à partir des données de continuité de quantité de mouvement des yeux portable et des données de continuité de quantité de mouvement de la tête portable.

14. Procédé de traitement de données d'électro-oculographie compatible avec un dispositif portable selon la revendication 13, comprenant par ailleurs le fait de:

acquérir, à partir des données de continuité d'électro-oculographie portable et des données de continuité de mouvement de la tête portable, les données de continuité de mouvement coordonné de la tête et des yeux portable continues relatives à un mouvement coordonné du mouvement des yeux et du mouvement de la tête tandis que le dispositif portable (1, 100) est utilisé, et

sortir les données de continuité de quantité de mouvement de ligne de vision portable qui correspondent aux données de continuité de mouvement coordonné de la tête et des yeux portable prédéterminées.

FIG.1

EP 3 662 813 B1

EP 3 662 813 B1

FIG.2

| ELECTROOCULOGRAPHY MEASURING DEVICE | WEARABLE-DEVICE-COMPATIBLE-ELECTROOCULOGRAPHY-DATA-PROCESSING DEVICE |
|---|---|
| 30 | WEARABLE-ELECTROOCULOGRAPHY-CONTINUITY-DATA-ACQUISITION SECTION → WEARABLE-EYE-MOVEMENT-AMOUNT-CONTINUITY-DATA-ACQUISITION SECTION |

FIG.3

FIG.4

FIG.5

```
          ┌─────────────┐
          │    START    │
          └──────┬──────┘
                 │
                 ▼
  ┌──────────────────────────────┐
  │     MEASURE EYE POTENTIAL     │─── S1
  └──────────────┬───────────────┘
                 │
                 ▼
  ┌──────────────────────────────┐
  │ ACQUIRE WEARABLE-ELECTROOCULOGRAPHY- │─── S2
  │        CONTINUITY DATA        │
  └──────────────┬───────────────┘
                 │
                 ▼
  ┌──────────────────────────────┐
  │ ACQUIRE WEARABLE-EYE-MOVEMENT-AMOUNT- │─── S3
  │        CONTINUITY DATA        │
  └──────────────┬───────────────┘
                 │
                 ▼
          ┌─────────────┐
          │     END     │
          └─────────────┘
```

## FIG.6

```
START
```

ACQUIRE WEARABLE-ELECTROOCULOGRAPHY-
CONTINUITY DATA IN EACH VISUAL LINE DIRECTION
(0-DEGREE DIRECTION, 90-DEGREE DIRECTION,
180-DEGREE DIRECTION, 270-DEGREE DIRECTION) — SA1

ACQUIRE WEARABLE-EYE-MOVEMENT-AMOUNT-
CONTINUITY DATA IN EACH VISUAL LINE DIRECTION
(0-DEGREE DIRECTION, 90-DEGREE DIRECTION,
180-DEGREE DIRECTION, 270-DEGREE DIRECTION) — SA2

CALCULATE RESPECTIVE MEDIANS FROM
ACQUIRED WEARABLE-EYE-MOVEMENT-AMOUNT-
CONTINUITY DATA — SA3

CREATE COORDINATE SYSTEM IN
WEARABLE-EYE-MOVEMENT-AMOUNT-
CONTINUITY DATA USING MEDIANS — SA4

CORRECT ORIGIN OF COORDINATE SYSTEM — SA5

PERFORM AFFINE TRANSFORMATION
ON COORDINATE SYSTEM — SA6

CORRECT WEARABLE-EYE-MOVEMENT-AMOUNT-
CONTINUITY DATA — SA7

```
END
```

FIG.7

FIG.8

VISUAL LINE DIRECTION
90 DEGREES

◆ LATERAL
DIRECTION

■ LONGITUDINAL
DIRECTION

VISUAL LINE
DIRECTION
0 DEGREES

180 DEGREES

EYE MOVEMENT AMOUNT (°)

EYE MOVEMENT AMOUNT (°)

270 DEGREES

FIG.9

FIG.10

FIG.11

## FIG.12

```
START
```

ACQUIRE WEARABLE-ELECTROOCULOGRAPHY-
CONTINUITY DATA — SB1

EXTRACT WEARABLE-ELECTROOCULOGRAPHY-
CONTINUITY DATA IN PREDETERMINED PERIOD — SB2

CALCULATE AVERAGE VALUE OF WEARABLE-
ELECTROOCULOGRAPHY-CONTINUITY DATA IN
PREDETERMINED PERIOD — SB3

IS DIFFERENCE BETWEEN AVERAGE VALUE
OF WEARABLE-ELECTROOCULOGRAPHY-CONTINUITY DATA
AND REFERENCE VALUE LARGER THAN
PREDETERMINED VALUE? — SB4

NO

YES

OFFSET SUCH THAT AVERAGE VALUE OF
WEARABLE-ELECTROOCULOGRAPHY-
CONTINUITY DATA IS REFERENCE VALUE — SB5

```
END
```

FIG.13

# FIG.14

```
      ┌─────────────┐
      │    START    │
      └─────────────┘
             │
             ▼
┌───────────────────────────────────┐
│ ACQUIRE WEARABLE-ELECTROOCULOGRAPHY-│──── SC1
│         CONTINUITY DATA            │
└───────────────────────────────────┘
             │
             ▼
┌───────────────────────────────────┐
│ CALCULATE AVERAGE VALUE OF WEARABLE-│
│ ELECTROOCULOGRAPHY-CONTINUITY DATA IN│──── SC2
│      EACH PREDETERMINED PERIOD     │
└───────────────────────────────────┘
             │
             ▼
```

SC3

IS DIFFERENCE BETWEEN AVERAGE VALUE
OF WEARABLE-ELECTROOCULOGRAPHY-CONTINUITY DATA
AND REFERENCE VALUE LARGER THAN
PREDETERMINED VALUE?

NO

YES

SC4

OFFSET SUCH THAT AVERAGE VALUE OF
WEARABLE-ELECTROOCULOGRAPHY-
CONTINUITY DATA IS REFERENCE VALUE

```
      ┌─────────────┐
      │     END     │
      └─────────────┘
```

FIG.15

# FIG.16

```
          ┌──────────────┐
          │    START     │
          └──────┬───────┘
                 │
                 ▼
    ┌──────────────────────────────────┐
    │  ACQUIRE DISTRIBUTION OF WEARABLE-│ ── SD1
    │  EYE-MOVEMENT-AMOUNT-CONTINUITY DATA│
    └──────────────┬───────────────────┘
                   │
                   ▼
    ┌──────────────────────────────────┐
    │ CALCULATE DIFFERENCE BETWEEN INTERSECTION│ ── SD2
    │   OF WEARABLE-EYE-MOVEMENT-AMOUNT-│
    │ CONTINUITY DATA AND REFERENCE VALUE│
    └──────────────┬───────────────────┘
                   │
                   ▼
```

SD3

IS DIFFERENCE BETWEEN INTERSECTION
OF WEARABLE-EYE-MOVEMENT-AMOUNT-CONTINUITY DATA
AND REFERENCE VALUE LARGER THAN
PREDETERMINED VALUE?

NO

YES

SD4

PERFORM AFFINE TRANSFORMATION OF
WEARABLE-EYE-MOVEMENT-AMOUNT-
CONTINUITY DATA

END

EYE MOVEMENT AMOUNT
IN VERTICAL DIRECTION

EYE MOVEMENT AMOUNT
IN HORIZONTAL DIRECTION

PO

O

TO

FIG.17

EYE MOVEMENT AMOUNT
IN VERTICAL DIRECTION

EYE MOVEMENT AMOUNT
IN HORIZONTAL DIRECTION

PO'

O

TO

FIG.18

FIG.19

FIG.20

ELECTROOCULOGRAPHY MEASURING DEVICE

INERTIAL SENSOR

WEARABLE-DEVICE-COMPATIBLE-ELECTROOCULOGRAPHY-DATA-PROCESSING DEVICE

WEARABLE-ELECTROOCULOGRAPHY-CONTINUITY-DATA-ACQUISITION SECTION

WEARABLE-HEAD-MOTION-CONTINUITY-DATA-ACQUISITION SECTION

CONTINUITY-DATA-ARITHMETIC SECTION

WEARABLE-HEAD-EYE-COORDINATED-MOTION-CONTINUITY-DATA-ACQUISITION SECTION

WEARABLE-VISUAL-LINE-MOVEMENT-CONTINUITY-DATA-ACQUISITION SECTION

EP 3 662 813 B1

## FIG.21

START

ACQUIRE MEASURED DATA —SE1

SPECIFY EYE MOVEMENT AND HEAD MOTION —SE2

ASSOCIATE EYE MOVEMENT AND HEAD MOTION —SE3

CALCULATE VISUAL-LINE-MOVEMENT AMOUNT —SE4

DETERMINE PRECEDING MOTION —SE5

EXTRACT ACQUIRED DATA
IN PREDETERMINED PERIOD —SE6

CALCULATE HEAD-MOTION-PRECEDING RATIO —SE7

CALCULATE VISUAL-LINE-MOVEMENT
AMOUNT CORRESPONDING
TO HEAD-MOTION-PRECEDING RATIO —SE8

END

## FIG.22

| EYE POTENTIAL VR \\ EYE POTENTIAL VL | 0[mv]OR MORE AND LESS THAN 0.5[mv] | 0.5[mv]OR MORE AND LESS THAN 1.0[mv] | 1.0[mv]OR MORE AND LESS THAN 1.5[mv] | 1.5[mv]OR MORE AND LESS THAN 2.0[mv] | ... |
|---|---|---|---|---|---|
| 0[mv]OR MORE AND LESS THAN 0.5[mv] | 0 | −0.5 | −1.0 | −1.5 | ... |
| 0.5[mv]OR MORE AND LESS THAN 1.0[mv] | 0.5 | 0.0 | −0.5 | −1.0 | ... |
| 1.0[mv]OR MORE AND LESS THAN 1.5[mv] | 1.0 | 0.5 | 0.0 | −0.5 | ... |
| 1.5[mv]OR MORE AND LESS THAN 2.0[mv] | 1.5 | 1.0 | 0.5 | 0.0 | ... |
| ... | ... | ... | ... | ... | ... |

EP 3 662 813 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017070602 A
- US 2017150897 A1

- US 20040070729 A1

**Non-patent literature cited in the description**

- **SOICHIRO YOKOO.** Evaluation of Driver's Safety Margin Using Parameters Related to EOG and Head Movements for Wearable Device. Human Interface Society, 06 September 2016